Europäisches Patentamt

(19) European Patent Office

Office européen des brevets

(11) Publication number: **0 116 411**
**B1**

(12) **EUROPEAN PATENT SPECIFICATION**

(45) Date of publication of patent specification: **16.05.90**

(21) Application number: **84300184.3**

(22) Date of filing: **12.01.84**

(51) Int. Cl.⁵: **C 12 N 15/00,** C 12 P 21/02, C 07 H 21/04, C 12 N 1/20 // C12R1/125, C12R1/465, C12R1/19

(54) DNA expression vectors useful in bacillus cells.

(30) Priority: **18.01.83 US 458792**

(43) Date of publication of application:
**22.08.84 Bulletin 84/34**

(45) Publication of the grant of the patent:
**16.05.90 Bulletin 90/20**

(84) Designated Contracting States:
**BE CH DE FR GB IT LI LU NL SE**

(56) References cited:
**EP-A-0 063 494**

**MOLECULAR AND GENERAL GENETICS, vol. 186, no. 3, 1982, pages 339-346; C.P. MORAN et al.: "Nucleotide sequences that signal the initiation of transcription and translation in bacillus subtilis"**

**NATURE, vol. 293, no. 5830, September 1981, pages 309-311, Macmillan Journals Ltd. Chesham, Bucks, GB; D.S. GOLDFARB et al.: "Expression of Tn9-derived chloramphenicol resistance in bacillus subtilis"**

(73) Proprietor: **ELI LILLY AND COMPANY**
**307, East McCarty Street**
**Indianapolis Indiana 46285 (US)**

(72) Inventor: **Kovacevic, Steven**
**5620 Broadway**
**Indianapolis Indiana 46220 (US)**
Inventor: **Miller, James Robert**
**9244 Thrushwood Lane**
**Indianapolis Indiana 46250 (US)**
Inventor: **Hsiung, Hansen Maxwell**
**108 West 88th Street**
**Indianapolis Indiana 46260 (US)**

(74) Representative: **Hudson, Christopher Mark et al**
**Erl Wood Manor**
**Windlesham Surrey GU20 6PH (GB)**

Courier Press, Leamington Spa, England.

**Description**

The present invention relates to recombinant DNA expression vectors which comprise a *Bacillus subtilis* ribosome binding site-encoding DNA fragment ligated both to the *veg* promoter of *B. subtilis* and also to a gene that encodes a functional polypeptide. The invention also relates to transformants of the aforementioned vectors and to a method for producing a functional polypeptide wherein the polypeptide is produced in *Bacillus* and secreted into the growth medium.

The expression vectors provided by the present inventions are for use in *Bacillus* and other host cells. Heretofore, the development and exploitation of recombinant DNA technology in *Bacillus* has been retarded and made especially difficult because of the general lack of suitable cloning and expression vectors. This paucity of expression vectors is explained in part by the lack of recognition afforded foreign transcription and translation initiation signals in *Bacillus*. Consequently, the well known *trp* (Hallewell, R. A. and S. Emtage, 1980, Gene 9:27), *lac* (Guarante, L. *et al.,* 1980, Cell 20:543 and Roberts, T. M. *et al.,* 1979, Proc. Nat. Acad. Sci. USA 76:5596), *1pp* (Lee, N. *et al.,*1981, J. of Bacteriol. 146:861; Zwiebel, L. J. *et al.,* 1981, J. of Bacteriol. 145:654 and Natamura, K. and M. Inouye, 1979, Cell 18:1109) and Bacteriophage $\lambda P_L$ (Derom, C. *et al.,* 1982, Gene 17:45; Remaut, E. *et al.,* 1981, Gene 15(1):81 and Bernard, H. *et al.,* 1979, Gene 5:59) transcription and translation-directing promoter systems are not functional in *Bacillus*. Thus, with the exception of a few drug resistance genes from gram positive organisms such as *Staphylococcus* and *Streptococcus,* few foreign and practically no eukaryotic genes have been expressed in *Bacillus*.

The extremely limited ability of *Bacillus* to recognize foreign transcription and translation signals necessitates the development of endogenous signals that direct gene expression. Several early cloning attempts include the cloning and expression of the *B. licheniformis* beta-lactamase gene, (disclosed in European Patent Office Publication (of European Patent Application No. 81300858.8) No. 0036259) and the *B. stearothermophilus* and *B. amyloliquefaciens* α-amylase genes, (respectively disclosed in European Patent Office Publication (of European Patent Application No. 82300158.1) No. 0057976 and Derwent Abstract (of Belgium Patent Application No. BE 891—659) No 37323 E/19) in *B. subtilis*. In addition, the *veg* promoter and translation signals (endogenous to *B. subtilis* and disclosed in Moran Jr., C. P. *et al.,* 1982, Mol. Gen. Genet. 186:339), have also been isolated and are useful as a starting material for purposes of constructing the present invention. Accordingly, the aforementioned *veg* promoter- and translation signal-containing sequence was modified and engineered to be useful for directing the expression of virtually any polypeptide in *Bacillus*. This represents a significant advance in the technical art and helps fill the need for expression vectors which are useful in gram positive microorganisms.

Gene cloning and expression of products in *Bacillus subtilis* are highly advantageous since the organism is non-pathogenic, does not produce endotoxins and can secrete gene products into the growth medium. In addition, *B. subtilis* has been extensively studied and is the archetype for genetic studies among gram positive microorganisms. The small and versatile expression vectors of the present invention are particularly important because they allow for the commercial exploitation of these important advantages.

For purposes of the present invention, as disclosed and claimed herein, the following terms are as defined below.

Recombinant DNA Expression Vector — any autonomously replicating agent, including but not limited to plasmids, comprising a DNA molecule to which one or more additional DNA segments can or have been added.

Transformation — the introduction of DNA into a recipient host cell that changes the genotype and consequently results in a change in the recipient cell.

Transformant — a recipient host cell that has undergone transformation.

Restriction Fragment — any linear portion or whole of plasmid or chromosomal DNA generated by the action of one or more restriction enzymes.

Functional Polypeptide — a recoverable bioactive entirely heterologous polypeptide or precursor, a recoverable bioactive polypeptide comprising a heterologous polypeptide and a portion or whole of a homologous polypeptide, or a recoverable bioinactive fusion polypeptide comprising a heterologous polypeptide and bioinactivating homologous polypeptide which can be specifically cleaved.

Fused Gene Product — a recoverable heterologous polypeptide which is fused with a portion or whole of a homologous polypeptide.

Insertional Isomer — one of the two or more possible recombinant DNA molecules formed when a DNA fragment is inserted at one of two or more compatible sites on the recipient DNA.

The present invention specifically provides a process for preparing recombinant DNA expression vectors which comprises ligating

1) the ribosome binding site-containing DNA sequence

```
5'  A G T G A G G T G G A  T  R  C                3'
        I  I  I  I  I  I  I  I  I  I
3'              T C C A C C T A R  G  G  T  A  C  5'
```

wherein
A is deoxyadenyl,
G is deoxyguanyl,
C is deoxycytosyl,
T is thymidyl,
R is G or C, and
$R^1$ is G or C,
to

2) the *veg* promoter of *Bacillus subtilis*, and
3) a gene that encodes a functional polypeptide,
subject to the limitation that R and $R^1$ are not simultaneously the same deoxyribonucleotide and subject to the further limitation that said vector is selectable and that said promoter and said DNA sequence direct transcription and expression of said gene in a host cell transformed by said vector. The invention further comprises transformants of the aforementioned vectors and also a method for producing a functional polypeptide wherein the polypeptide is produced in *Bacillus* and secreted into the culture medium.

The ribosome binding site-containing DNA sequence to which the *veg* promoter and gene are ligated can be conventionally synthesized by the modified phosphotriester method, using fully protected trideoxyribonucleotide building blocks, in substantial accordance with the procedures of Itakura *et al.,* 1977, Science 198:1056 and Crea *et al.,* 1978, Proc Nat. Acad. Sci. USA 75:5765. The *veg* promoter can either be synthesized directly or obtained by *Eco*RI-*Sfa*NI digestion of plasmid pMS480. The resultant ~.38 kb *Eco*RI-*Sfa*NI fragment contains the *veg* promoter as well as additional deoxyribonucleotides at the 5' end of the coding strand (adjacent to the *Eco*RI sticky terminus). Plasmid pMS480 is ~4.8 kb and can be conventionally isolated from *E. coli* K12 JA221/pMS480, a strain deposited and made part of the permanent stock culture collection of the Northern Regional Research Laboratory, Peoria, Illinois. The strain is available to the public as a preferred source and stock reservoir of plasmid pMS480 under the accession number NRRL B—15258.

For convenience and ease of construction, the *veg* promoter was obtained by *Eco*RI-*Sfa*NI digestion of plasmid pMS480. The resultant fragment was then ligated to the aforedescribed ribosome binding site-containing DNA sequence, a sequence designed to have *Sfa*NI and *Nco*I sticky ends. The sequence thus allows for the direct expression of a polypeptide upon simultaneous ligation with both a *Nco*I-restricted gene and the aforementioned ~.38 kb *Eco*RI-*Sfa*NI *veg* promoter-containing fragment. Direct expression results because the ligation of the *Nco*I sticky ends restores the ATG translational start triplet of the *Nco*I-restricted gene. The synthetic sequence is therefore useful for the universal direct expression, under the control of the *Bacillus subtilis veg* promoter, of any gene that encodes a functional polypeptide.

Although genes that naturally contain a *Nco*I site at the translational start point are preferred, genes lacking such sites can also be used. In the latter case, the gene can be cleaved by a restriction enzyme and then reconstructed synthetically (Itakura *et al.,* 1977 and Crea *et al.,* 1978) so as to contain the desired *Nco*I sticky end. Alternatively, depending upon convenience and ease of construction, the modified gene may be entirely synthetic. In either case, the modified gene can be ligated to the *Nco*I sticky end of the aforementioned ribosome binding site-containing sequence thus restoring the ATG methionine-encoding start triplet and thus allowing for the direct expression of a desired product. The *Bacillus* promoter-containing expression vectors of the present invention represent a significant technical advance. They are universally applicable in *Bacillus* and can be used for the expression, under the control of a homologous *Bacillus* promoter, of any polypeptide-encoding gene.

Expression vectors illustrative of the present invention were constructed by ligating the ~.38 kb *Eco*RI-*Sfa*NI fragment of plasmid pMS480, the ~4 kb *Eco*RI-*Nco*I fragment of the pre-proinsulin plasmid pOW601 and the aforementioned ribosome binding site-containing DNA sequence. The resultant plasmid, designated as pOW10, is functional in *E. coli* and comprises a functional polypeptide-encoding gene in translational reading phase with the *veg* promoter. Plasmid pOW10 is particularly useful for constructing illustrative expression vectors that are functional in *Bacillus*. Plasmid pOW601, which is used as a starting material for constructing plasmid pOW10, is ~4.4 kb and can be conventionally isolated from *E. coli* K12 JA221/pOW601, a strain deposited and made part of the permanent stock culture collection of the Northern Regional Research Laboratory, Peoria, Illinois. The strain is available to the public as a preferred source and stock reservoir of the plasmid under the accession number NRRL B—15259.

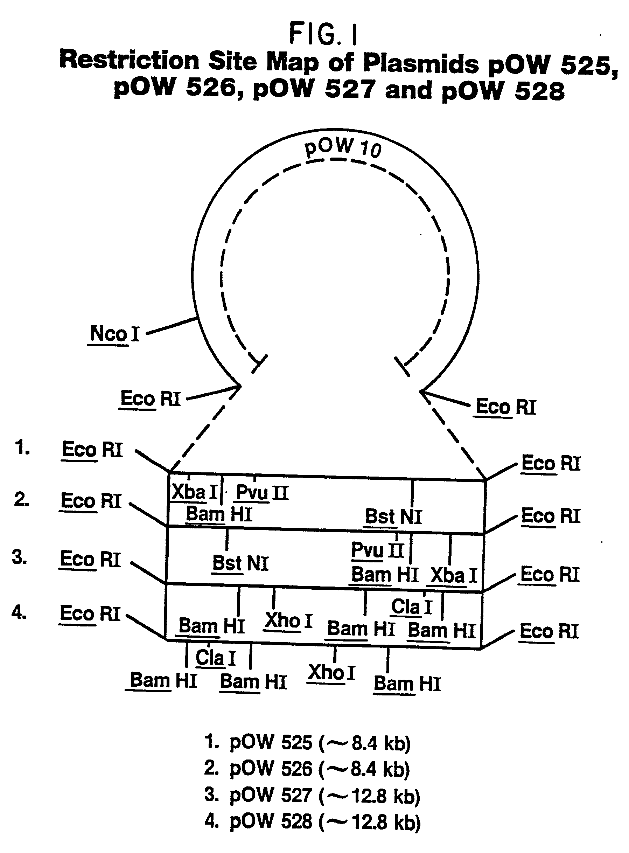

Illustrative vectors that are functional in *Bacillus* are constructed by ligating *Eco*RI-digested plasmid pOW10 into *Eco*RI-digested plasmid pHI-18 or pBS1. Plasmid pHI-18 is ~3.9 kb and contains a chloramphenicol resistance gene as well as an origin of replication that is functional in *Bacillus*. Plasmid pHI-18 is constructed by an ~.7 kb *Hpa*II deletion of plasmid pHI-16. The latter plasmid is an *in vivo* deletion of known chimeric plasmid pBD12 (disclosed in Gryczan *et al.,* 1980, J. Bacteriology 141(1):246) which can be conventionally isolated from *Bacillus subtilis* MI112/pHI-16, a constructed strain deposited and made part of the permanent stock culture collection of the Northern Regional Research Laboratory, Peoria, Illinois. The strain is available to the public as a preferred source and stock reservoir of the plasmid under the accession number NRRL B—12597. The aforementioned ligation into plasmid pHI-18 results in the illustrative ~8.4 kb plasmids pOW525 and pOW526. A restriction site map of each of the plasmids pOW525 and pOW526 is presented in Figure 1 of the accompanying drawings.

Ligation of *Eco*RI-digested plasmid pOW10 into *Eco*RI-digested plasmid pBS1 results in the illustrative ~12.8 kb plasmids pOW527 and pOW528. Plasmid pBS1 is constructed by ligating the ~4.6 kb *Bam*HI fragment of plasmid pHI-18 into the ~4.4 kb *Bam*HI fragment of plasmid pEL105. Plasmid pEL105 is constructed by ligating the ~1.6 kb *Bam*HI fragment of plasmid pLR2 (constructed by ligating *Hind*III-digested plasmid pIJ6 (disclosed in Thompson *et al.,* 1980, Nature 286:525), and *Hind*III-digested plasmid pBR322), into the ~2.8 kb *Bam*HI fragment of plasmid pEL103. The latter plasmid can be conventionally isolated from *Streptomyces granuloruber* No. A39912.13/pEL103, a strain deposited and made part of the permanent stock culture collection of the Northern Regional Research Laboratory, Peoria, Illinois. The strain is available as a preferred source and stock reservoir of the plasmid under the accession number NRRL 12549. A restriction site map of each of plasmids pOW527 and pOW528 is presented in Figure 1 of the accompanying drawings.

Plasmids pOW525, pOW526, pOW527 and pOW528 are functional in *Bacillus*, comprise a functional pre-proinsulin-encoding gene in translational reading phase with the *veg* promoter and ribosome binding site-containing synthetic DNA sequence and therefore exemplify the present invention. Other illustrative vectors were constructed by 1) digesting plasmid pOW10 with *Nco*I and plasmid pMC1403 with *Bam*HI restriction enzyme; 2) filling in the resulting sticky ends with the Klenow fragment of DNA polymerase; 3) digesting the filled-in fragments with *Eco*RI restriction enzyme and 4) ligating the resultant two fragments at their respective *Eco*RI and blunted ends. Plasmid pMC1403, which is used as a starting material for these constructions, is ~9.9 kb and contains a portion of the *lacZ* gene. The plasmid can be conventionally isolated from *E. coli* K12 BE904/pMC1403, a strain deposited and made part of the permanent stock culture collection of the Northern Regional Research Laboratory, Peoria, Illinois. The strain is available to the public as a preferred source and stock reservoir of the plasmid under the accession number NRRL B—15213. The aforementioned ligation restores both the *Nco*I and *Bam*HI restriction sites and therefore results in a plasmid, designated as plasmid pOW303, which contains a portion of the *lacZ* gene in translational reading phase with the *veg* promoter and ribosome binding site-containing synthetic DNA sequence.

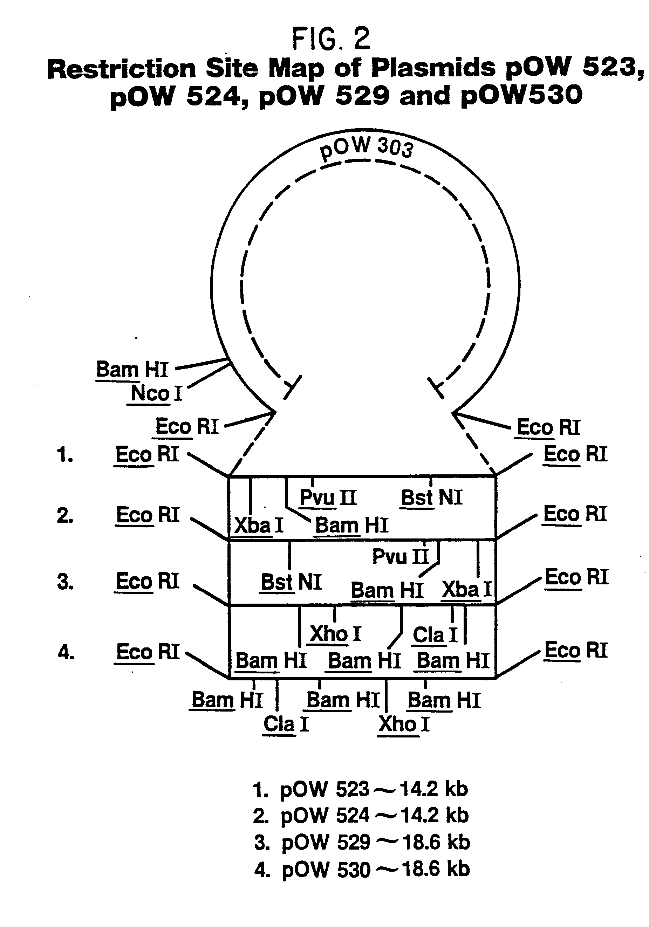

Plasmid pOW303 was digested with *Eco*RI restriction enzyme and ligated to *Eco*RI-digested plasmid pHI-18 to produce the illustrative plasmids pOW523 and pOW524. A similar construction, involving the substitution of *Eco*RI-digested plasmid pBS1 for the *Eco*RI-digested plasmid pHI-18, results in illustrative plasmids pOW529 and pOW530. Plasmids pOW523, pOW524, pOW529 and pOW530 are functional in *Bacillus*, comprise a functional polypeptide-encoding gene in translational reading phase with the *veg* promoter and aforementioned DNA sequence and therefore further illustrate the present invention. The β-galactosidase activity conferred to host cells by the aforementioned vectors can be employed as a selectable marker making the vectors generally useful for molecular cloning. Restriction site maps of plasmids pOW523, pOW524, pOW529 and pOW530 are presented in Figure 2 of the accompanying drawings.

The present invention also provides a method for producing a functional polypeptide in *Bacillus* wherein said polypeptide is secreted into the culture medium. Secretion occurs when the polypeptide-encoding gene of the present invention also encodes a signal peptide. Signal peptides are short leader regions of amino acids which often comprise newly synthesized polypeptides and which are believed to function in the transport of polypeptides across cell membranes. Signal peptides are typically cleaved from the newly synthesized polypeptides during transport. The aforementioned method for secretion, which is in no way limited by the underlying transport mechanism, comprises transforming a *Bacillus* host cell with a recombinant DNA expression vector which comprises

1) the ribosome binding site-containing DNA sequence

$$5' \; A \; G \; T \; G \; A \; G \; G \; T \; G \; G \; A \; T \; R \; C \; \quad 3'$$
$$| \; | \; | \; | \; | \; | \; | \; | \; |_1| $$
$$3' \quad\quad T \; C \; C \; A \; C \; C \; T \; A \; R^1 G \; G \; T \; A \; C \; \; 5'$$

wherein
A is deoxyadenyl,
G is deoxyguanyl,
C is deoxycytosyl,
T is thymidyl,
R is G or C, and
$R^1$ is G or C,
2) the *veg* promoter of *Bacillus subtilis,* and
3) a gene that encodes a signal peptide-containing functional polypeptide,
and culturing said transformed *Bacillus* host cell under growth conditions, subject to the limitation that R and $R^1$ are not simultaneously the same deoxyribonucleotide and subject to the further limitation that said vector is selectable and that said promoter and said DNA sequence direct transcription and expression of said gene in said transformed *Bacillus* cell.

Plasmids pOW525, pOW526, pOW527 and pOW528, and the *Bacillus subtilis* MI112 transformants thereof, exemplify the present method for secretion. The plasmids each comprise the aforedescribed *veg*

promoter, ribosome binding site-containing DNA sequence and a gene that encodes pre-proinsulin, the signal peptide-containing form of proinsulin. When *Bacillus subtilis* host cells are transformed by these vectors, pre-proinsulin is produced intracellularly and proinsulin is secreted into the culture medium.

The aforementioned method for secretion is not limited to the use of genes encoding pre-proinsulin. Any gene that codes for a signal peptide-containing functional polypeptide can be used including, for example, genes that code for immune modulators, pre-growth hormone, pre-human growth hormone, pre-porcine growth hormone, proteolytic degradative enzymes and cellulolytic degradative enzymes. Moreover, DNA encoding a given signal peptide can be synthesized directly or cleaved from pre-existing genes and then ligated to genes that normally lack such signal peptide-encoding sequences. In this way, any gene that encodes a functional polypeptide can be modified such that the present method for secretion can be applied. Thus, the present method is not limited to the use of genes that naturally contain a signal peptide-encoding region.

The present invention is particularly versatile and can be applied to the production of any polypeptide which can be encoded by a gene in a recombinant DNA cloning vector. A preferred recombinant DNA cloning vector is the plasmid although bacteriophage and other vectors can also be used and are apparent to those skilled in the art. In addition to the illustrative pre-proinsulin and *lacZ* genes, other genes that can be used include genes that are naturally occurring, genes that are non-naturally occurring and genes that are in part naturally occurring and in part synthetic or non-naturally occurring. More particularly, the genes can code for human proinsulin, human insulin A-chain, human insulin B-chain, non-human insulin, human growth hormone, non-human growth hormone, bovine growth hormone, porcine growth hormone, human interferon, non-human interferon, viral antigen, urokinase, any peptide hormone, any enzyme or virtually any other polypeptide with research or commercial value.

The recombinant DNA expression vectors of the present invention are not limited for use in a single species or strain. To the contrary, the vectors are broadly applicable and can be transformed into host cells of many taxa, particularly the restrictionless strains of *Bacillus, Streptomyces* and *E. coli*. Restrictionless strains are readily selected and isolated from *Bacillus* and *Streptomyces* taxa by conventional procedures and extensions of principles well known in the art (Lomovskaya *et al.,* 1980, Microbiological Reviews 44:206). Host cells of restrictionless strains lack restriction enzymes and therefore do not cut or degrade plasmid DNA upon transformation. For purposes of the present application, host cells containing restriction enzymes that do not cut any of the restriction sites of the present vectors are also considered restrictionless.

Preferred host cells of restrictionless strains of *Bacillus*, in which the present vectors are especially useful and can be transformed, include restrictionless cells of, for example, *B. subtilis, B. subtilis* MI112, *B. thuringiensis, B. thuringiensis* var. *israeliensis, B. cereus, B. anthracis, B. piliformis, B. tropicus, B. alvei, B. megaterium, B. pumilus, B. licheniformis, B. polymyxa, B. macerans, B. circulans, B. stearothermophilus, B. coagulans, B. firmus, B. brevis, B. sphaericus, B. pasteurii, B. fastidiosus, B. larvae, B. lentimorbus, B. apiarus, B. amyloliquifaciens, B. laterosporus,* and *B. popillae.*

Preferred host cells of restrictionless strains of *Streptomyces* taxa that produce aminoglycoside antibiotics and in which the present vectors are especially useful and can be transformed, include restrictionless cells of, for example: *S. kanamyceticus* (kanamycins), *S. chrestomyceticus* (aminosidine), *S. griseoflavus* (antibiotic MA 1267), *S. microsporeus* (antibiotic SF—767), *S. ribosidificus* (antibiotic SF733), *S. flavopersicus* (spectinomycin), *S. spectabilis* (actinospectacin), *S. rimosus* forma *paromomycinus* (paromomycins, catenulin), *S. fradiae* var. *italicus* (aminosidine), *S. bluensis* var. *bluensis* (bluensomycin), *S. catenulae* (catenulin), *S. olivoreticuli* var. *cellulophilus* (destomycin A), *S. tenebrarius* (tobramycin, apramycin), *S. lavendulae* (neomycin), *S. albogriseolus* (neomycins), *S. albus* var. *metamycinus* (metamycin), *S. hygroscopicus* var. *sagamiensis* (spectinomycin), *S. bikiniensis* (streptomycin), *S. griseus* (streptomycin, *S. erythrochromogenes* var. *narutoensis* (streptomycin), *S. poolensis* (streptomycin), *S. galbus* (streptomycin), *S. rameus* (streptomycin), *S. olivaceus* (streptomycin), *S. mashuensis* (streptomycin), *S. hygroscopicus* var. *limoneus* (validamycins), *S. rimofaciens* (destomycins), *S. hygroscopicus* forma *glebosus* (glebomycin), *S. fradiae* (hybrimycins neomycins), *S. eurocidicus* (antibiotic A16316—C), *S. aquacanus* (N-methyl hygromycin B), *S. crystallinus* (hygromycin A), *S. noboritoensis* (hygromycin), *S. hygroscopicus* (hygromycins), *S. atrofaciens* (hygromycin), *S. kasugaspinus* (kasugamycins), *S. kasugaensis* (kasugamycins), *S. netropsis* (antibiotic LL—AM31), *S. lividus* (lividomycins), *S. hofuensis*(seldomycin complex), and *S. canus* (ribosyl paromamine).

Preferred host cells of restrictionless strains of *Steptomyces* taxa that produce macrolide antibiotics and in which the present vectors are especially useful and can be transformed, include restrictionless cells of, for example: *S. caelestis* (antibiotic M188), *S. platensis* (platenomycin), *S. rochei* var. *volubilis* (antibiotic T2636), *S. venezuelae* (methymycins), *S. griseofuscus* (bundlin), *S. narbonensis* (josamycin, narbomycin), *S. fungicidicus* (antibiotic NA—181), *S. griseofaciens* (antibiotic PA133A, B), *S. roseocitreus* (albocycline), *S. bruneogriseus* (albocycline), *S. roseochromogenes* (albocycline), *S. cinerochromogenes* (cineromycin B), *S. albus* (albomycetin), *S. felleus* (argomycin, picromycin), *S. rochei* (lankacidin, borrelidin), *S. violaceoniger* (lankacidin), *S. griseus* (borrelidin), *S. maizeus* (ingramycin), *S. albus* var. *coilmyceticus* (coleimycin), *S. mycarofaciens* (acetyl-leukomycin, espinomycin), *S. hygroscopicus* (turimycin, relomycin, maridomycin, tylosin, carbomycin), *S. griseospiralis* (relomycin), *S. lavendulae* (aldgamycin), *S. rimosus* (neutramycin), *S. delta* (deltamycins), *S. fungicidicus* var. *espinomyceticus* (espinomycins), *S. furdicidicus*

(mydecamycin), *S. ambofaciens* (foromacidin D), *S. eurocidicus* (methymycin), *S. griseolus* (griseomycin), *S. flavochromogenes* (amaromycin, shincomycins), *S. fimbriatus* (amaromycin), *S. fasciculus* (amaromycin), *S. erythreus* (erythromycins), *S. antibioticus* (oleandomycin), *S. olivochromogenes* (oleandomycin), *S. spinichromogenes* var. *suragaoensis* (kujimycins), *S. kitasatoensis* (leucomycin), *S. narbonensis* var. *josamyceticus* (leucomycin A3, josamycin), *S. albogriseolus* (mikonomycin), *S. bikiniensis* (chalcomycin), *S. cirratus* (cirramycin), S. djakartensis (niddamycin), *S. eurythermus* (angolamycin), *S. fradiae* (tylosin, lactenocin, macrocin), *S. goshikiensis* (bandamycin), *S. griseoflavus* (acumycin), *S. halstedii* (carbomycin), *S. tendae* (carbomycin), *S. macrosporeus* (carbomycin), *S. thermotolerans* (carbomycin), and *S. albireticuli* (carbomycin).

Preferred host cells of restrictionless strains of *Streptomyces* taxa that produce β-lactam antibiotics and in which the present vectors are especially useful and can be transformed, include restrictionless cells of, for example: *S. lipmanii* (A16884, MM4550, MM13902), *S. clavuligerus* (A16886B, clavulanic acid), *S. lactamdurans* (cephamycin C), *S. griseus* (cephamycin A, B), *S. hygroscopicus* (deacetoxycephalosporin C), *S. wadayamensis* (WS—3442—D), *S. chartreusis* (SF 1623), *S. heteromorphus* and *S. panayensis* (C2081X); *S. cinnamonensis, S. fimbriatus, S. halstedii, S. rochei* and *S. viridochromogenes* (cephamycins A, B); *S. cattleya* (thienamycin); and *S. olivaceus, S. flavovirens, S. flavus, S. fulvoviridis, S. argenteolus,* and *S. sioyaensis* (MM 4550 and MM 13902).

Preferred host cells of restrictionless strains of *Streptomyces* taxa that produce polyether antibiotics and in which the present vectors are especially useful and can be transformed, include restrictionless cells of, for example: *S. albus* (A204, A28695A and B, salinomycin), *S. hygroscopicus* (A218, emericid, DE3936), A120A, A28695A and B, etheromycin, dianemycin), *S. griseus* (grisorixin), *S. conglobatus* (ionomycin), *S. eurocidicus* var. *asterocidicus* (laidlomycin), *S. lasaliensis* (lasalocid), *S. ribosidificus* (lonomycin), *S. cacaoi* var. *aseoensis* (lysocellin), *S. cinnamonensis* (monensin), *S. aureofaciens* (narasin), *S. gallinarius* (RP 30504), *S. longwoodensis* (lysocellin), *S. flaveolus* (CP38936), *S. mutabilis* (S—11743a), and *S. violaceoniger* (nigericin).

Preferred host cells of restrictionless strains of *Streptomyces* taxa that produce glycopeptide antibiotics and in which the present vectors are especially useful and can be transformed, include restrictionless cells of, for example: *S. orientalis* and *S. haranomachiensis* (vancomycin); *S. candidus* (A—35512, avoparcin), and *S. eburosporeus* (LL—AM 374).

Preferred host cells of other *Streptomyces* restrictionless strains in which the present vectors are especially useful and can be transformed, include restrictionless cells of, for example: *S. coelicolor, S. granuloruber, S. roseosporus, S. lividans, S. espinosus* and *S. azureus.*

The invention is not limited for use in *Bacillus* and *Streptomyces* but can also be used in various *E. coli* host cells. Preferred *E. coli* host cells include *E. coli* K12, *E. coli* K12 JA221, *E. coli* K12 HB101, *E. coli* K12 C600, *E. coli* K12 C600$M_k^-R_k^-$, *E. coli* K12 C600$M_k^+R_k^-$ and *E. coli* K12 RV308.

While all the embodiments of the present invention are useful, some of the present expression vectors are preferred. Accordingly, preferred vectors are plasmids pOW523, pOW524, pOW525, pOW526, pOW527, pOW528, pOW529, and pOW530 and preferred transformants are *Bacillus subtilis* MI112/pOW523, *B. subtilis* MI112/pOW524, *B. subtilis* MI112/pOW525, *B. subtilis* MI112/pOW526, *B. subtilis* MI112/pOW527, *B. subtilis* MI112/pOW528, *B. subtilis* MI112/pOW529 and *B. subtilis* MI112/pOW530. Of this preferred group, plasmids pOW523, pOW525, pOW527 and pOW529 and transformants *B. subtilis* MI112/pOW523, *B. subtilis* MI112/pOW525, *B. subtilis* MI112/pOW527 and *B. subtilis* MI112/pOW529 are most preferred.

The recombinant DNA expression vectors and transformants of the present invention have broad utility and help fill the need for expression vehicles, especially for use in *Bacillus*. Thus, the present vectors allow for the genetic expression and secretion in *Bacillus* of products now bioproduced in *E. coli*. This is especially advantageous because large scale fermentation of *Bacillus* is better known and understood than is fermentation of *E. coli*. In fact, commercial fermentation of *E. coli* is still highly experimental and fraught with difficulty. The present invention circumvents this problem by providing the alternative of producing compounds now biosynthesized in *E. coli* such as, for example, human insulin, human proinsulin, glucagon, interferon, human growth hormone, bovine growth hormone and the like, in *Bacillus*. This can be done because the present vectors are highly versatile and can accommodate DNA sequences which encode the aforementioned products. Thus, the present invention allows for flexibility in the choice of hosts and provides a means for using *Bacillus* in the bioproduction of polypeptides and other gene products.

The ability of the present transformants to secrete polypeptide products is commercially advantageous. For example, isolation and purification of polypeptides can be done continuously during fermentation without the lytic destruction of host cells. Secretion also affords protection against proteolytic degradation of gene products by naturally occurring protease enzymes. Microorganisms are notorious for producing such enzymes which rapidly digest unprotected foreign polypeptides. The present method for secretion circumvents this problem by providing a means for removing susceptible polypeptides from the host cell before proteolytic degradation can occur. In addition, host cells are also protected from the toxic effects of a given gene product since secretion out of the cell prevents the deleterious effects and possible cell death associated with intracellular build-up.

*Streptomyces granuloruber* No. A39912.13/pEL103, *Bacillus subtilis*/MI112/pHI-16, *E. coli* K12 JA221/ pMS480, *E. coli* K12 BE904/pMC1403 and *E. coli* K12 JA221/pOW601, as respective sources of plasmids

pEL103, pHI-16, pMS480, pMC1403 and pOW601, and *Streptomyces ambofaciens* can be cultured in a number of ways using any of several different media. Carbohydrate sources which are preferred in a culture medium include, for example, molasses, glucose, dextrin, and glycerol, and nitrogen sources include, for example, soy flour, amino acid mixtures, and peptones. Nutrient inorganic salts are also incorporated and include the customary salts capable of yielding sodium, potassium, ammonium, calcium, phosphate, chloride, sulfate, and like ions. As is necessary for the growth and development of other microorganisms, essential trace elements are also added. Such trace elements are commonly supplied as impurities incidental to the addition of other constituents of the medium.

*Streptomyces granuloruber* No. A39912.13/pEL103 is grown under aerobic culture conditions over a relatively wide pH range of about 5 to 9 at temperatures ranging from about 15° to 40°C. For production of plasmid pEL103 in the greatest quantity, however, it is desirable to start with a culture medium at a pH of about 7.2 and maintain a culture temperature of about 30°C. Culturing *Streptomyces granuloruber* No. A39912.13/pEL103, under the aforementioned conditions, results in a reservoir of cells from which plasmid pEL103 is isolated conveniently by techniques well known in the art.

*Bacillus subtilis* MI112/pHI-16 is grown under aerobic culture conditions over a relatively wide pH range of about 5 to 8.5 at temperatures ranging from about 25° to 45°C. For production of plasmid pHI-16 in the greatest quantity, however, it is desirable to start with a culture medium at a pH of about 7 and maintain a culture temperature of about 37°C. Culturing *Bacillus subtilis* MI112/pHI-16, under the aforementioned conditions, results in a reservoir of cells from which plasmid pHI-16 is isolated conveniently by techniques well known in the art.

*E. coli* K12 JA221/pMS480, *E. coli* K12 BE904/pMC1403 and *E. coli* K12 JA221/pOW601 are each grown under aerobic culture conditions over a relatively wide pH range of about 6.5 to 8 at temperatures ranging from about 25° to 40°C. For production of plasmids pMS480, pMC1403 and pOW601 in the greatest quantity, however, it is desirable to start with a culture medium at a pH of about 7.2 and maintain a culture temperature of about 37°C. Culturing the *E. coli* cells, under the aforementioned conditions, results in a reservoir of cells from the plasmids are respectively isolated by techniques well known in the art.

The following examples further illustrate and detail the invention disclosed herein. Both an explanation of and the actual procedures for constructing the invention are described where appropriate.

## Example 1

Construction of Plasmid pOW10

A. Construction of the ~.38 kb *Eco*RI-*Sfa*NI Fragment of Plasmid pM480

1. Isolation of Plasmid pMS480

The bacterium *E. coli* K12 JA221/pMS480 (NRRL B-15258) was cultured in TY broth (1% tryptone, .5% yeast extract, .5% sodium chloride, pH 7) with 100 µg./ml. of antibiotic ampicillin according to conventional microbiological procedures. After 18 hours incubation, about .5 ml. of the culture was transferred to a 1.5 ml. Eppendorf tube and centrifuged for about 15 seconds. Unless otherwise indicated, all the manipulations were done at ambient temperature. The resultant supernatant was carefully removed with a fine-tip aspirator and the cell pellet was suspended in about 100 µl. of freshly prepared lysozyme solution which contained 2 mg./ml. lysozyme, 50 mM glucose, 10 mM EDTA (ethylene diaminetetracetate) and 25 mM Tris-HCl (pH 8). After incubation at 0°C. for 30 minutes, about 200 µl. of alkaline SDS (sodium dodecyl sulfate) solution (.2N NaOH), 1% SDS) were added and then the tube was gently votexed and maintained at 0°C. for 5 minutes. Next, about 150 µl. of 3 M sodium acetate (prepared by dissolving 3 moles of sodium acetate in a minimum of water, adjusting the pH to 4.8 with glacial acetic acid and then adjusting the volume to 1 l.) were added a DNA clot formed after the contents of the tube were mixed gently for a few seconds by inversion.

The tube was maintained at 0°C. for 60 minutes and then centrifuged for 5 minutes to yield an almost clear supernatant. About .4 ml. of the supernatant was transferred to a second centrifuge tube to which 1 ml. of cold ethanol was added. After the tube was held at −20°C. for 30 minutes, the resultant precipitate was collected by centrifugation (2 minutes) and the supernatant was removed by aspiration. The thus collected pellet was dissolved in 200 µl. of .1 M sodium acetate/.05 M Tris-HCl (pH 8) and was reprecipitated by the addition of 2 volumes of cold ethanol. After 10 minutes at −20°C., the precipitate was collected by centrifugation and constituted the desired plasmid pMS480 DNA.

2. *Eco*RI-*Sfa*NI Digestion of Plasmid pMS480

About 5 μl. (5 μg.) of plasmid pMS480 (isolated in Example A—1) in TE buffer (10 mM Tris-HCl, pH 8., 1 mM EDTA), 5 μl. DTT (100 mM Dithiothreitol), 5 μl. (1000 μg./ml.) BSA (bovine serum albumin), 25 μl. water, 5 μl. (5 New England Biolab units) *Eco*RI restriction enzyme* and 5 μl. 10X reaction mix** were incubated at 37°C. for about 1 hour. The reaction was terminated by incubation at 65°C. for 10 minutes. Next, the reaction mixture was cooled on ice and then about 1.1 μl. of 5 M NaCl, 4 μl. water and 5 μl. (5 New England Bio lab units) *Sfa*NI restriction enzyme were added followed by a second incubation at 37°C. for 1 hour. The reaction was terminated by incubation at 65°C. for 10 minutes and then the reaction mixture was cooled on ice, extracted with each of phenol and chloroform:isoamyl alcohol (24:1) and then ethanol precipitated. The desired ~.38 kb *Eco*RI-*Sfa*NI restriction fragments were conventionally separated and isolated by agarose gel electrophoresis (Maniatis *et al.,* 1982, Molecular Cloning, Cold Spring Harbor Laboratory, Cold Spring Harbor, New York). The desired ~.38 kb fragments were dissolved in about 30 μl. of water.

B. Construction of the ~4 kb *Eco*RI-*Nco*I Fragment of Plasmid pOW601

1. Isolation of Plasmid pOW601

Plasmid pOW601 is isolated from *E. coli* K12 JA221/pOW601 (NRRL B—15259). The strain was cultured and the plasmid was isolated in substantial accordance with the teaching of Example 1A—1.

2. *Eco*RI-*Nco*I Digestion of Plasmid pOW601

The desired digestion is carried out in substantial accordance with the teaching of Example 1A—2 except that plasmid pOW601, rather than plasmid pMS480, was used.

C. Construction of the DNA Fragment

$$
\begin{array}{l}
5'\ A\ G\ T\ G\ A\ G\ G\ T\ G\ G\ A\ T\ R\ C \qquad\qquad 3'\\
\quad\ |\ \ |\ \ |\ \ |\ \ |\ \ |\ \ |\ \ |\ \ |_{1}\,|\\
3' \qquad\quad T\ C\ C\ A\ C\ C\ T\ A\ R^{1}G\ G\ T\ A\ C\ \ 5'
\end{array}
$$

wherein R is G and $R^1$ is C

The desired construction involves the synthesis and 5' phosphorylation of oligonucleotides $T_1$ and $T_2$ shown below.

| | | |
|---|---|---|
| $T_1$ 5' | AGTGAGGTGGATGC | 3' |
| $T_2$ 5' | CATGGCATCCACCT | 3' |

Oligonucleotides $T_1$ and $T_2$ were used to construct the desired DNA fragment having an *Nco*I sticky terminus. The oligonucleotide synthesis was conventionally done by the modified phosphotriester method using fully protected deoxyribonucleotide building blocks.

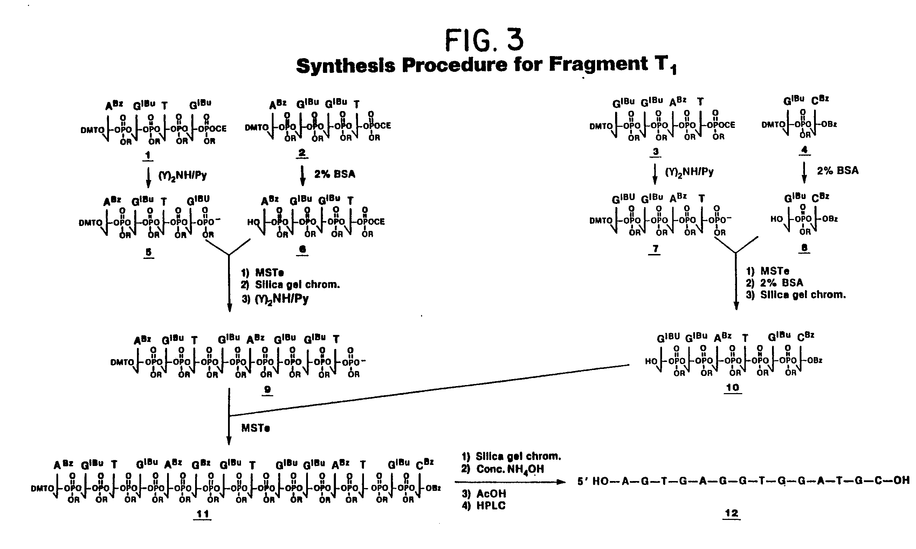

The above synthesis is typified by the following procedure for fragment $T_1$ as summarized in Figure 3 of the accompanying drawings. Various nucleotide fragments that are used in the synthesis of $T_1$ are numerically designated in the figure. The abbreviations employed are as follows: MSTe, mesitylenesulfonyltetrazole; BSA, benzene sulfonic acid; TLC, thin layer chromatography; HPLC, high performance liquid chromatography; DMT, 4,4'-dimethoxytrityl; CE, 2-cyanoethyl; R, p-chlorophenyl; Bz, benzoyl; $(Y)_2$NH, diisopropylamine; iBu, isobutyryl; Py, pyridine; AcOH, acetic acid.

The fully protected deoxyribotetranucleotides *2* (312 mg., .15 mmol) and deoxyribodinucleotides *4* (50 mg., .017 mmol) are deblocked at the 5' hydroxyls by treatment with 2% BSA in 7:3 (v/v) chloroform/methanol (2 and 1 ml., respectively) for 10 minutes at 0°C. Reactions are stopped by addition of saturated aqueous ammonium bicarbonate (2 ml.), extracted with chloroform (25 ml.), and washed with water (2x, 10 ml.). The organic layers are dried (magnesium sulfate), concentrated to small volumes (about 5 ml.), and precipitated by addition of petroleum ether (35°—60°C. fraction). The colorless precipitates are collected by centrifugation and dried in a dessicator *in vacuo* to give *6* and *8*, respectively, each homogeneous by silica gel tlc (Merck 60 F254, chloroform/methanol, 9:1).

Tetramers *1* and *3* (400 mg., .17 mmol; 100 mg., .043 mmol) are converted into their phosphodiesters (*5* and *6*) by treatment with diisopropylamine/pyridine (1:5, v/v, 2 ml.) for 30 minutes at ambient temperature. The reaction mixture was then precipitated with the addition of anhydrous ether (20 ml.).

---

*Restriction and other enzymes can be obtained from the following sources:

New England Bio Labs., Inc., 32 Tozer Road, Beverly, Massachusetts 01915

Boehringer-Mannheim Biochemicals, 7941 Castleway Drive, Indianapolis, Indiana 46250

**Reaction mix (10X) for *Eco*RI restriction enzyme was prepared with the following composition:

500 mM NaCl

1000 mM Tris-HCl, pH 7.2

50 mM $MgCl_2$

Reagents are removed by centrifugation and decanting the supernatant. The residues are dried by evaporation with anhydrous pyridine. Dimer 8 (.04 mmol) and tetramer 7 are combined with MSTe (65 mg., .25 mmol) in anhydrous pyridine (1 ml.) and the reaction mixture left at ambient temperature for two hours. TLC analysis shows that 95% of the dimer 8 has been converted into hexamer product (visualized by detection of the DMT group by spraying with 10% aqueous sulfuric acid and heating at 100°C.). The reaction mixture is precipitated by addition of ether and the supernatant is decanted. The hexamer is deblocked at the 5' position with 2% BSA (8 ml.) as described above for dimer 4 and tetramer 2. The product (10) is purified on a silica gel column (Merck 60 H, 3.5 × 5 cm.) by step gradient elution with chloroform/methanol (98:2 to 95:5, v/v). Fractions containing product 10 are evaporated to dryness.

Similarly, tetramer 5 is coupled to tetramer 6 and the fully protected product directly purified on silica gel. This latter compound is deblocked at the 3' end by diisopropylamine/pyridine as described above to give fragment 9.

Finally, octomer 9 and hexamer 10 are coupled in anhydrous pyridine (.5 ml.) with MSTe (100 mg., .39 mmol) as the condensing agent. Upon completion (45 minutes, ambient temperature) the mixture is rotary evaporated and the residue chromatographed on silica gel. A portion of compound 11 (20 mg.) in pyridine (.5 ml.) is completely deblocked by treatment with concentrated ammonium hydroxide (7 ml., 8 hours, 60°C.) and subsequent treatment in 80% acetic acid (15 minutes, ambient temperature). After evaporation of acetic acid, the solid residue is dissolved in distilled water (2 ml.) and extracted with ethyl ether (3x, 2 ml.). The aqueous phase is concentrated to dryness and redissolved in 50% pyridine/water. The product was purified by preparative thin layer chromatography on Polyethyleneimine-Cellulose (PEI/UV$_{254}$) plates (Narang S.A. et al., 1980, Methods in Enzymology 65:610) and then by HPLC on a reverse phase C—18 column (Waters). The sequence of 12 is confirmed by two-dimensional sequence analysis.

Next, oligonucleotide T$_2$ was constructed. This was done in substantial accordance with the above synthesis protocol for oligonucleotide T$_1$.

Ten microgram quantities of the resultant oligonucleotide T$_1$ and T$_2$ are quantitatively phosphorylated with [γ-$^{32}$P]-ATP (New England Nuclear) in the presence of T$_4$ polynucleotide kinase to give specific activities of approximately 1 Ci/mmol. Radiolabelled fragments are purified by 20% polyacrylamide/7M urea gel electrophoresis and sequences of the eluted fragments are verified by two-dimensional electrophoresis/homochromatography (Jay et al., 1974, Nucleic acids Res. 1:331) of partial snake venom digests. Fragments T$_1$ and T$_2$ are then conventionally annealed to form the desired DNA fragment.

D. Construction of Plasmid pOW10 by ligation of 1) the ~.38 kb EcoRI-SfaNI Fragment of Plasmid pMS480; 2) the ~4 kb EcoRI-NcoI Fragment of Plasmid pOW601 and 3) the Synthetic DNA Fragment of Example 1C and Construction of E. coli K12 JA221/pOW10.

About 8 µl. (.01 µg.) of the ~.38 kb EcoRI-SfaNI fragment of plasmid pMS480, 8 µl. (.10 µg.) of the ~4 kb EcoRI-NcoI fragment of plasmid pOW601, 10 µl. (.7 µg.) of the DNA fragment of Example 1C, 10 µl. water, 4 µl. (10 mM) ATP, 2.5 µl. (100 mM) dithiothreitol (DDT), 5 µl. ligation mix** and 2.5 µl. T4 DNA ligase* (~2 New England Bio Lab Units) were incubated at 16°C. for about 16 hours. The reaction was terminated by incubation at 65°C. for 10 minutes and then, after cooling on ice, the resultant ligated mixture was used to transform E. coli K12 JA221 (NRRL B—15211), in substantial accordance with the transformation procedure of Lederberg and Cohen, 1974, J. Bacteriology 119:1072, on TY plate containing 10 µg./ml. of antibiotic tetracycline. The resultant transformants were conventionally cultured and used for subsequent production and isolation of plasmid pOW10 in substantial accordance with the procedure of Example 1A. A restriction site map of plasmid pOW10 is shown in Figure 1 of the accompanying drawings.

Example 2
Construction of Plasmids pOW525 and pOW526 and E. coli K12 JA221/pOW525 and E. coli K12 JA221/pOW526
A. Isolation of Plasmid pHl-16
1. Culture of Bacillus subtilis Ml112/pHl-16

A vegetative culture of Bacillus subtilis Ml112/pHl-16 (NRRL B—12597) was conventionally prepared by plating on PAB agar (PAB† [Penassay broth] containing agar at 15 g./l. and chloramphenicol at 10 µg./ml.). After the inoculated plate was incubated at 37°C. for about 18 hours, a single colony was selected and used for inoculating 500 ml. of sterilized PAB medium with 10 µg./ml. chloramphenicol. The resultant inoculated broth was incubated at 37°C. for about 18 hours after which the resultant Bacillus subtilis Ml112/pHl-16 cells were ready for harvest and subsequent isolation of plasmid DNA.

---

*T4 DNA ligase can be obtained from the following source:
    New England Bio Labs., Inc., 32 Tozer Rd., Beverly, Massachusetts 01915
**Ligation mix was prepared with the following composition:
    500 mM Tris-HCl, pH 7.6
    100 mM MgCl$_2$
†PAB can be obtained from Difco Laboratories, Detroit, Michigan.

2. Plasmid Isolation

About 10 g. (wet wgt) of *Bacillus subtilis* MI112/pHI-16 cells were first harvested by centrifugation (10 minutes, 4°C., 10,000 rpm), then washed in about 50 ml. TES (10 mM Tris (pH 8), 10 mM NaCl, 1 mM EDTA) and finally collected again by centrifugation. About 20 ml. TE buffer with 25% sucrose were added to the pellet followed by about 10 mg. of lysozyme in 250 µl. water. The mixture was incubated at 37°C. for about 30 minutes followed by the addition of about 100 units of RNase. The resultant mixture was again incubated at 37°C. for 30 minutes and then, upon being made 1% and 1 M with respect to SDS (sodium dodecyl sulfate) and sodium chloride respectively, the mixture was cooled in an ice bath for about 3 hours. After the lysate was centrifuged (30 minutes, 4°C., 19,000 rpm), the supernatant was adjusted to 31.8 ml. with TE, and then 28.7 g. of cesium chloride and .4 ml. (10 mg./ml.) of ethidium bromide were added. A cesium chloride gradient was established by centrifuging at 49,500 rpm for 16 hours. The plasmid band was collected and centrifuged at 55,000 rpm for 16 hours, then collected again, extracted thrice with equal volumes of isoamyl alcohol, dialyzed against dilute TE, ethanol precipitated, and resuspended in 400 µl. of TE. The resultant plasmid pHI-16 DNA was stored at 4°C. for future use.

The kanamycin resistance gene is contained within the ~.74 kb *Hpa*II fragment of plasmid pHI-16. Therefore, treatment with *Hpa*II restriction enzyme followed by ligation results in a ~3.9 kb plasmid, designated herein as pHI-18, which lacks the kanamycin resistance gene. A detailed procedure for constructing plasmid pHI-18 is described below.

B. Construction of Plasmid pHI-18
1. Partial *Hpa*II Digestion of Plasmid pHI-16

About 5 µl. (2.5 µg.) of plasmid pHI-16 DNA, 1 µl. (2 mg./ml.) BSA, 37 µl. water, 2 µl. of *Hpa*II (containing 2 New England Bio Labs units) restriction enzyme, and 5 µl. reaction mix* were incubated at 37°C. for 1 hour. After the reaction was terminated by heating at 65°C. for 10 minutes, the DNA was precipitated by adding 2 volumes of 95% ethanol. The resultant DNA precipitate was washed in 70% ethanol, dried *in vacuo*, suspended in 5 µl. of TE buffer, and stored at 4°C. for future use.

2. Ligation of Plasmid pHI-16 *Hpa*II Digest

About 5 µl. of plasmid pHI-16 *Hpa*II digest (prepared in Example 3B—1), 2 µl. T4 DNA ligase, and 43 µl. ligation mix† were incubated at about 16°C. for about 18 hours. The reaction was terminated by the addition of about 5 µl. of 3M sodium acetate and 150 µl. of 95% ethanol. The desired DNA precipitate was washed in 70% ethanol, dried *in vacuo*, suspended in 10 µl. of TE buffer, and stored at 4°C. for future use.

C. Construction of *Bacillus subtilis* MI112/pHI-18

*Bacillus subtilis* MI112 can be obtained by conventionally culturing *B. subtilis* MI112/pHI-16 (NRRL B—12597) in the absence of chloramphenicol. The *B. subtilis* MI112/pHI-16 cells spontaneously lose the pHI-16 plasmid under the aforementioned culture conditions thus generating the desired chloramphenicol sensitive *B. subtilis* MI112 strain. Those skilled in the art will recognise and understand that sensitivity to chloramphenicol can be employed for testing and insuring that only *B. subtilis* MI112 cells that lack the plasmid are selected and used in the *Bacillus* transformation procedures herein disclosed.

About 50 ml. of sterile PAB was inoculated with *Bacillus subtilis* MI112 and incubated at 37°C. until a cell density of $2 \times 10^8$ cells/ml. was reached. The cells were then protoplasted, using sterile technique, by pelleting and then resuspending the cells in about 5 ml. of SMMP (equal volumes of each of 4x PAB and a solution comprising 1.0 M sucrose, .04 M maleic acid, and .04 M $MgCl_2$, pH adjusted to 6.5 with NaOH). Next, about 250 µl. of lysozyme (20 mg./ml. in SMM [0.5 M sucrose, .02 M maleic acid, and .02 M $MgCl_2$, pH adjusted to 6.5 with NaOH]) were added using filter sterilization. The cells were incubated with gentle shaking at 37°C. for about 2 hours. The resultant protoplasts were pelleted, washed with 5 ml. SMMP, and then resuspended in 5 ml. SMMP. Following centrifugation (25°C., 12 minutes, 2,600 rpm), about .1 ml. of the protoplasts were transformed by adding about 20 µl. of a 1:1 mixture comprising plasmid pHI-18 DNA and 2X SMM. About 1.5 ml. of PEG solution (40 g. PEG 6000 [polyethyleneglycol], 50 ml. 2X SMM, and water to 100 ml.) were then immediately added and, after about 2 minutes, 5 ml. of SMMP were also added. Next, the protoplasts were pelleted, suspended in 1 ml. of SMMP, and incubated at 30°C. with gentle

---

*Reaction mix for *Hpa*II restriction enzyme was prepared with the following composition.
  60 mM KCl
  100 mM Tris-HCl, pH 7.4
  100 mM $MgCl_2$
  10 mM Dithiothreitol

†Ligation mix was prepared with the following composition.
  66   mM Tris-HCl, pH 7.8
  10   mM Dithiothreitol
  6.6 mM $MgCl_2$
  4   mM ATP

shaking for about 2 hours. Aliquots of the thus prepared suspension were plated on chloramphenicol containing DM3 regeneration medium which per liter had the following composition.

91 g. D-mannitol in 555 ml. deionized water containing 12 g. agar
10% Casamino acids 50 ml.
10% Yeast extract 50 ml.
20% Glucose 25 ml.
5% Dipotassium phosphate 100 ml.
1 M $MgCl_2$ 20 ml.
10% Gelatin
10 mg Chloramphenicol

The D-mannitol, casamino acids and yeast extract were autoclaved together. The gelatin was added immediately after autoclaving and the remaining ingredients were added after the mixture had cooled. The medium had a final chloramphenicol concentration of 10 µg./ml.

The resultant chloramphenicol resistant colonies were tested for kanamycin sensitivity. A chloramphenicol resistant and kanamycin sensitive colony was selected as the desired *Bacillus subtilis* MI112/pHI-18 strain. The strain was cultured and the identity further confirmed by conventional restriction enzyme and agarose gel electrophoretic analysis (Maniatis *et al.,* 1982), of the constitutive plasmid.

D. *Eco*RI Digestion of Plasmids pHI-18 and pOW10

About 4 µl. (3 µg.) of pHI-18, 1 µl. (1 mg.) of pOW10, 5 µl. water, 1 µl. 10X *Eco*RI buffer and 1.5 µl. (containing 5 New England Bio Lab units) *Eco*RI restriction enzyme were incubated at 37°C. for 1.5 hours. After the reaction was terminated by incubation at 65°C. for 10 minutes, the *Eco*RI-digested DNA was cooled on ice, ethanol precipitated and then dissolved in 30 µl. of water.

E. Ligation of the Plasmid pHI-18 and pOW10 *Eco*RI Digests and Construction of *E. coli* K12 JA221/pOW525 and *E. coli* K12 JA221/pOW526

About 30 µl. of the pHI-18 and pOW10 *Eco*RI digests, 4 µl. (100 mM) 10X Dithiothreitol, 4 µl. (10 mM) ATP, 4 µl. 10X ligase buffer and 1 µl. (containing 3 New England Bio Lab units) T4 DNA ligase were incubated at 16°C. for about 4 hours. The reaction was terminated by incubation at 65°C. for 10 minutes and then, after cooling on ice, the resultant ligated DNA was used to transform *E. coli* K12 JA221 (NRRL B—15211), in substantial accordance with the transformation procedure of Lederberg and Cohen (1974), on TY plates containing 10 µg./ml of tetracycline. The resultant transformants were conventionally cultured and used for subsequent production and isolation of plasmids pOW525 and pOW526 in substantial accordance with the procedure of Example 1A. Plasmids pOW525 and pOW526 can be conventionally identified and distinguished by restriction enzyme and agarose gel electrophoretic analysis (Maniatis *et al.,* 1982). A restriction site map of each of plasmids pOW525 and pOW526 is presented in Figure 1 of the accompanying drawings.

Example 3
Construction of *Bacillus subtilis* MI112/pOW525 and *B. subtilis* MI112/pOW526

The desired constructions were made in substantial accordance with the procedure of Example 2C except that plasmids pOW525 and pOW526, rather than plasmid pHI-18, were used. The resultant *Bacillus subtilis* MI112/pOW525 and *B. subtilis* MI112/pOW526 chloramphenicol-resistant and kanamycin-sensitive transformant colonies are isolated, according to known procedures, and then conventionally identified by restriction enzyme and agarose gel electrophoretic analysis of the constitutive plasmids. The thus constructed transformants, useful for the subsequent production and isolation of plasmids pOW525 and pOW526, produce pre-proinsulin intracellularly and also secrete proinsulin into the culture medium. This was determined by *in vitro* assay of both cell lysates and the culture medium.

Example 4
Construction of Plasmids pOW527 and pOW528 and *E. coli* K12 JA221/pOW527 and *E. coli* K12 JA221/pOW528

The desired constructions were made in substantial accordance with the teaching of Example 2 except that plasmid pBS1, rather than plasmid pHI-18, was used. Plasmid pBS1 is constructed as outlined below.

A. Isolation of Plasmid pEL103

1. Culture of *Streptomyces granuloruber* No. A39912.13/pEL103

A vegetative inoculum of *Streptomyces granuloruber* No. A39912.13/pEL103 (NRRL 12549) was conventionally prepared by growing the strain under submerged aerobic conditions in 50 ml. of sterilized trypticase soy broth* at 35 g./l. in deionised water.

The trypticase soy broth inoculum was incubated for 48 hours at a temperature of 30°C. After incubation, about 10 ml. of the inoculum was transferred to 500 ml. of the sterilized broth and was incubated for about 20 hours at 30°C. The pH was not adjusted. After incubation, the *Streptomyces granuloruber* No. A39912.13/pEL103 cells were ready for harvest and subsequent isolation of plasmid DNA.

2. Plasmid Isolation

About 12 g. (wet wgt) of *Streptomyces granuloruber* No. A39912.13/pEL103 cells were centrifuged (10 minutes, 4°C., 10,000 rpm), washed in 10% glycerol, and then harvested by recentrifugation under the aforementioned conditions. About 50 ml. of TES Buffer (.01 M Tris(hydroxymethyl)aminoethane [Tris], .001 M EDTA, 34% sucrose, pH 8) were added to the cells followed by about 0.25 g. of lysozyme in 10 ml. of 0.25 M EDTA. After the mixture was incubated at 37°C. for about 15 minutes, about 0.5 ml. of 10% Triton X—100 in TE buffer (.01 M Tris, .001 M EDTA, pH 8) was added. The resultant mixture was then incubated at 65°C. for about 15 minutes. After the lysate was centrifuged (45 minutes, 4°C., 18,000 rpm), the supernatant was extracted four times with isoamyl alcohol and once with a chloroformisoamyl alcohol solution (24:1). Next, .1 volume of 3M sodium acetate was added to the aqueous phase followed by 3 volumes of cold (−20°C.) 95% ethanol. The ethanol precipitation was rapidly performed in a dry ice-ethanol bath and the DNA precipitate was collected by centrifugation (15 minutes, 4°C., 10,000 rpm). The precipitate was vacuum dried and then resuspended in 1.1 ml. of STE buffer (.01 M Tris, .001 M EDTA, .01 M sodium chloride). Centrifugation (40 hours, 15°C., 35,000 rpm) using cesium chloride gradients, with ethidium bromide, was carried out to purify the plasmid DNA. Following centrifugation, the desired plasmid pEL103 DNA band was removed and the ethidium bromide extracted by conventional procedures. After precipitation of the DNA in 3 volumes of ethanol, the thus isolated plasmid pEL103 DNA was dissolved in 1 ml. of 10 fold diluted TE buffer and was then stored at −20°C.

B. Construction of Plasmid pLR2

1. *Hind*III Digestion of Plasmid pIJ6

About 20 µl. (20 µg.) of plasmid pIJ6 DNA, disclosed in Thompson *et al.,* 1980, Nature 286:525, 5 µl. BSA (Bovine Serum albumin, 1 mg./ml.), 19 µl. water, 1 µl. of *Hind*III (containing 3 New England Bio Labs units) restriction enzyme, and 5 µl. reaction mix** were incubated at 37°C. for 2 hours. The reaction was terminated by the addition of about 50 µl. of 4M ammonium acetate and 200 µl. of 95% ethanol. The resultant DNA precipitate was washed twice in 70% ethanol, dried *in vacuo*, suspended in 20 µl. of TE buffer, and stored at −20°C.

2. *Hind*III Digestion of Plasmid pBR322

About 8 µl. (4 µg.) of plasmid pBR322 DNA†, 5 µl. reaction mix, 5 µl. BSA (1 mg./ml.), 31 µl. water, and 1 µl. of *Hind*III restriction enzyme were incubated at 37°C. for 2 hours. After the reaction was terminated by incubating at 60°C. for 10 minutes, about 50 µl. of 4 M ammonium acetate and 200 µl. of 95% ethanol were added. The resultant DNA precipitate was washed twice in 70% ethanol, dried *in vacuo*, and suspended in 45 µl. of water.

---

*Trypticase soy broth is obtained from BBL Division, Becton-Dickinson & Company, Cockeysville, Maryland 21030

**Reaction mix for *Hind*III restriction enzyme was prepared with the following composition.

600 mM NaCl

100 mM Tris-HCl, pH 7.9

70 mM $MgCl_2$

10 mM Dithiothreitol

†Plasmid pBR322 can be obtained from Boehringer-Mannheim Biochemicals the address of which is disclosed in Example 1A—2.

### 3. Ligation of HindIII Digested Plasmids pIJ6 and pBR322

About 20 µl. of *Hind*III treated plasmid pIJ6, 20 µl. of *Hind*III treated plasmid pBR322, 5 µl. BSA (1 mg./ ml.), 1 µl. of T4 DNA ligase, and 5 µl. ligation mix* were incubated at 16°C. for 4 hours. The reaction was terminated by the addition of about 50 µl. 4M ammonium acetate and 200 µl. of 95% ethanol. The resultant DNA precipitate was washed twice in 70% ethanol, dried *in vacuo,* and suspended in TE buffer. The suspended DNA constituted the desired plasmid pLR2.

### C. Construction of *E. Coli* K12 HB101/pLR2

About 10 ml. of *E. coli* K12 HB101 cells (Bolivar *et al.,* 1977, Gene 2:75—93) were pelleted by centrifugation and then suspended in about 10 ml. of 0.01M sodium chloride. Next, the cells were pelleted again, resuspended in about 10 ml. of 0.03M calcium chloride, incubated on ice for 20 minutes, pelleted a third time, and finally, resuspended in 1.25 ml. of 0.03M calcium chloride. The resultant cell suspension was competent for subsequent transformation.

Plasmid pLR2 in TE buffer was ethanol precipitated, suspended in 150 µl. of 30mM calcium chloride solution, and gently mixed in a test tube with about 200 µl. of competent *E. coli* K12 HB101 cells. The resultant mixture was incubated on ice for about 45 minutes and then at 42°C. for about 1 minute. Next, about 3 ml. of L-broth (Bertani, 1951, J. Bacteriology 62:293) containing 50 µg/ml. of ampicillin was added. The mixture was incubated with shaking at 37°C. for 1 hour and then plated on L-agar (Miller, 1972, Experiments in Molecular Genetics, Cold Spring Harbor Labs, Cold Spring Harbor, New York) containing ampicillin. Surviving colonies were selected and tested for the expected phenotype (Amp[R], Tet[S]), and constituted the desired *E. coli* K12 HB101/pLR2 transformants.

### D. Construction of Plasmids pEL107 and pEL105

#### 1. *Bam*HI Digestion of Plasmid pLR2 and Isolation of the ~1.6 kb Thiostrepton Resistance-Conferring Fragment

About 50 µg. of plasmid pLR2 DNA, 10 µl. reaction mix*, 10 µl. BSA (1 mg./ml.), 29 µl. water, and 1 µl. (4 units/µl.) of *Bam*HI restriction enzyme were incubated at 37°C. for 2 hours. After adding an equal volume of 4M ammonium acetate and 2.5 volumes of 95% ethanol, the mixture was cooled at −20°C. for about 18 hours to precipitate the DNA. The DNA precipitate was collected by centrifugation and then suspended in about 50 µl. of TE buffer. The desired ~1.6 kb *Bam*HI restriction fragment was isolated conventionally from the DNA suspension by agarose gel electrophoresis (Maniatis *et al.* 1982). Following isolation, the fragment was resuspended in about 20 µl. of TE buffer for subsequent ligation.

#### 2. Partial *Bam*HI Digestion of Plasmid pEL103

About 20 µg. of plasmid pEL103 DNA, 10 µl. reaction mix, 10µl. BSA (1 mg./ml.), 39 µl. water, and 1 µl. of *Bam*HI restriction enzyme (prepared by diluting 2 µl. of enzyme in 8 µl. of water) were incubated at ambient temperature for about 15 minutes. After adding an equal volume of 4M ammonium acetate and 2 volumes of 95% ethanol, the mixture was cooled at −20°C. for about 18 hours to precipitate the DNA. The DNA precipitate was collected by centrifugation, rinsed in 70% ethanol, dried *in vacuo,* and then suspended in about 50 µl. of TE buffer.

#### 3. Ligation

A mixture of about 20 µg. of the partially digested plasmid pEL103 DNA, 10 µg. of the ~1.6 kb *Bam*HI restriction fragment of plasmid pLR2, 5 µl. ligation mix, 5 µl. BSA (1 mg./ml.), 10 µl. water, and 1 µl. T4 DNA ligase were incubated at about 16°C. for about 4 hours. After adding 40 µl. of 4M ammonium acetate and 200 µl. of cold ethanol, the mixture was cooled to −20°C. for about 18 hours to precipitate the DNA. The DNA precipitate was collected by centrifugation, washed with 70% ethanol, collected again, and then suspended in 50 µl. of medium P (Hopwood and Wright 1978, Molecular and General Genetics 162:307) for subsequent transformation.

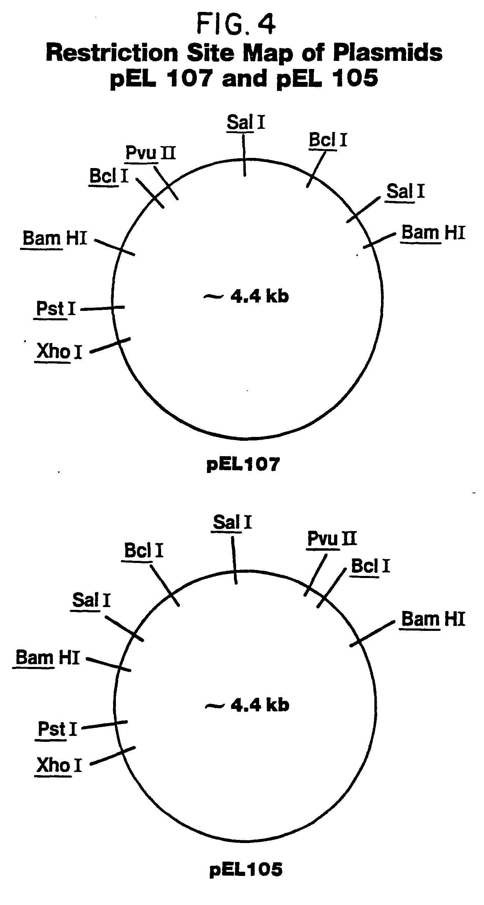

Recombinant plasmids of various types result depending upon which of the possible pEL103 restriction fragments becomes ligated to the ~1.6 kb *Bam*HI thiostrepton resistance-conferring fragment. Ligation to the ~2.8 kb BamHI restriction fragment of plasmid pEL103 results in the desired ~4.4 kb plasmids pEL107 and pEL105. Recombinant plasmids of two orientations result because the ~1.6 kb *Bam*HI resistance-conferring fragment can be oriented in either direction. A restriction site and functional map of each of plasmids pEL107 and pEL105 is presented in Figure 4 of the accompanying drawings.

---

* Ligation mix was prepared with the following composition.
500mM Tris-HCl, pH7.8
200mM Dithiothreitol
100mM $MgCl_2$
10mM ATP

E. Construction of Streptomyces ambofaciens/pEL107 and *S. ambofaciens*/pEL105

Using about 20 µg. of the DNA from Example 4D—3 and $1 \times 10^8$ protoplasts (prepared according to Baltz, 1978, J. of General Microbiology 107:93) of *Streptomyces ambofaciens,* a strain deposited and made part of the permanent stock culture collection of the Northern Regional Research Laboratory, Peoria, Illinois, from which it is available to the public under the accession number NRRL 2420, the desired constructions were made in substantial accordance with the teaching of International Publication (of International Patent Application No. PCT/GB79/00095) No. WO79/01169, Example 2. The desired transformants were selected for thiostrepton resistance by overlaying the regenerating protoplasts with modified R2 medium (Baltz, 1978, J. of General Microbiology 107:93) top agar containing sufficient thiostrepton to bring the final plate concentration to 50 µg/ml. The resultant *Streptomyces ambofaciens*/pEL107 and *S. ambofaciens*/pEL105 thiostrepton resistant colonies were isolated according to known procedures, cultured and then identified by restriction enzyme and gel electrophoretic analysis (Maniatis *et al.,* 1982), of the constitutive plasmids.

Accordingly, vegetative inocula (10 ml.) of different isolated colonies are conventionally prepared by inoculating trypticase soy broth containing sufficient thiostrepton to bring the final concentration to 50 µg./ ml. Several inocula are prepared and the following procedure performed until all the desired transformant types and constitutive plasmids are isolated. Thus, after cells are incubated at 30°C. until fully grown, 6 ml. of the cell-containing broth are centrifuged. The resultant pellet is washed in TE buffer, pelleted again, and then suspended in 400 µl. 50mM Tris, pH 8.0. Next, about 80 µl. of .25M EDTA, 20 µl. RNase, and 100 µl. (10 mg./ml. in TE) lysozome are added. After the mixture is incubated at 37°C. for about 15 minutes, about 10 µl. of 10% Triton X—100 and 150 µl. 5M NaCl are added followed by a final incubation at 60°C. for 15 minutes. The resultant lysate is centrifuged (15 minutes, 4°C., 15,000 rpm) and then the supernatant is conventionally extracted twice with phenol, once with a chloroform-isoamyl alcohol solution (24:1), and then ethanol precipitated. The identity of the constitutive plasmids and thus the transformants are determined conventionally by restriction enzyme and agarose gel electrophoretic analysis (Maniatis *et al.,* 1982). Plasmid pEL105 was conventionally isolated for subsequent construction of plasmid pBS1.

F. Construction of Plasmids pBS1 and pBS3
1. Partial *Bam*HI Digestion of Plasmid pEL105

About 10 µl. (5 µg.) of plasmid pEL105 (conventionally isolated from *Streptomyces ambofaciens*/pEL105 [prepared in Example 4E] in substantial accordance with the teaching of Example 4A—1), 2 µl. BSA (1 mg./ml.), 29 µl. water, 1 µl. of *Bam*HI (diluted 1:4 with water) restriction enzyme, and 5 µl. reaction mix were incubated at 25°C. for 15 minutes. The reaction was terminated by the addition of about 50 µl. of 4M ammonium acetate and 300 µl. of 95% ethanol. After cooling at −20°C. for about 2 hours, the resultant DNA precipitate was collected by centrifugation, washed twice in 70% ethanol, dried *in vacuo*, and then suspended in about 10 µl. of TE buffer. Because plasmid pEL105 has two *Bam*HI restriction sites, a mixture of different fragments results.

2. *Bam*HI Digestion of Plasmid pHI—18

About 5 µl. (5 µg.) of plasmid pHI—18, 2 µl. BSA (1 mg./ml.), 9 µl. water, 1 µl. of *Bam*HI (4 units/µl.) restriction enzyme, and 1.5 µl. reaction mix were incubated at 37°C. for about 2 hours. After adding an equal volume of 4M ammonium acetate and 2.5 volumes of 95% ethanol, the mixture was cooled at −20°C. for about 18 hours to precipitate the DNA. The DNA precipitate was collected by centrifugation, washed in 70% ethanol, and then suspended in about 10 µl. of TE buffer.

3. Ligation

About 5 µl. of *Bam*HI-digested plasmid pHI—18, 8 µl. of plasmid pEL105 *Bam*HI partial digest, 27 µl. water, 5 µl. (4mM) APT, 5 µl. ligation mix, and 2 µl. T4 DNA ligase were incubated at 16°C. for about 18 hours. The reaction was terminated by the addition of 50 µl. 4M ammonium acetate and 200 µl. of 95% ethanol. After incubation at −20°C. for about 2 hours, the desired plasmid pBS1 and pBS3 DNA precipitate was collected by centrifugation, washed in 70% ethanol, dried *in vacuo,* suspended in 10 µl. of TE buffer, and stored at 4°C. for future use.

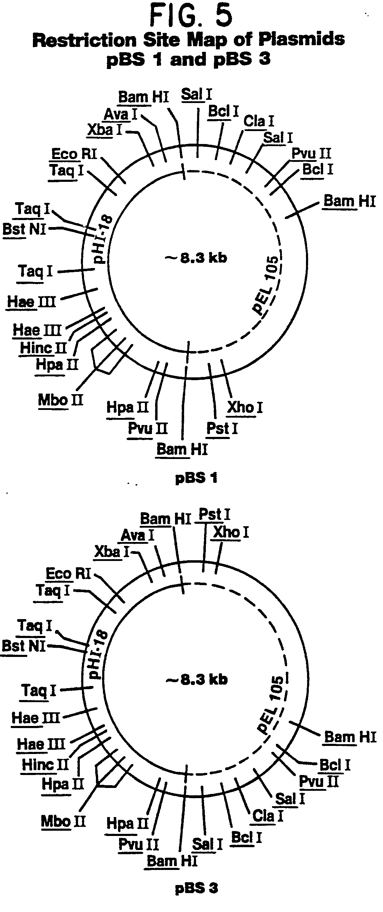

Since plasmid pEL105 has two *Bam*HI restriction sites, a partial *Bam*HI digest results in two ~4.4 kb *Bam*HI fragments. Therefore, the insertional isomers of plasmids pBS1 and pBS3 are also produced by the above procedure. Recombinant plasmids of two orientations result because the *Bam*HI restricted DNA can be ligated in either direction. A restriction site and functional map of each of plasmids pBS1 and pBS3 is presented in Figure 5 of the accompanying drawings.

G. Construction of *Bacillus subtilis* MI112/pBS1 and *B. subtilis* MI112/pBS3

The desired constructions were made in substantial accordance with the procedure of Example 2C except that plasmids pBS1 and pBS3, rather than plasmid pHI—18, were used. The resultant *Bacillus subtilis* MI112/pBS1 and *B. subtilis* MI112/pBS3 chloramphenicol resistant and kanamycin sensitive transformant colonies were isolated according to known procedures, cultured and then conventionally identified by restriction enzyme and agarose gel electrophoretic analysis (Maniatis *et al.,* 1982), of the constitutive plasmids. *Bacillus subtilis* MI112/pBS1 was selected and cultured for subsequent isolation of plasmid pBS1.

H. Final Construction of Plasmids pOW527 and pOW528 and *E. coli* K12 JA221/pOW527 and *E. coli* K12 JA221/pOW528

The desired plasmids pOW527 and pOW528 and transformants *E. coli K12* JA221/pOW527 and *E. coli* K12 JA221/pOW528 were constructed in substantial accordance with the procedure of Example 2 except that plasmid pBS1, rather than plasmid pHI—18, was used. Recombinant plasmids of two orientations result because the *Eco*RI-restricted DNA can be ligated in either direction. A restriction site map of each of plasmids pOW527 and pOW528 is presented in Figure 1 of the accompanying drawings.

Example 5
Construction of *Bacillus subtilis* MI112/pOW527 and *B. subtilis* MI112/pOW528

The desired constructions are made in substantial accordance with the teaching of Example 2C except that plasmids pOW527 and pOW528, rather than plasmid pHI—18, are used. The resultant *Bacillus subtilis* MI112/pOW527 and *B. subtilis* MI112/pOW528 chloramphenicol resistant and kanamycin sensitive transformant colonies are isolated, according to known procedures, and then conventionally identified by restriction enzyme and agarose gel electrophoretic analysis (Maniatis *et al.,* 1982), of the constitutive plasmids. The thus constructed transformants, useful for the subsequent production and isolation of plasmids pOW527 and pOW528, produce pre-proinsulin intracellularly and also secrete proinsulin into the culture medium. This was determined by *in vitro* assay of both cell lysates and the culture medium.

Example 6
Construction of Plasmid pOW303 and *E. coli* K12 BE904/pOW303

A. *Nco*I Digestion of Plasmid pOW10

About 2 µl. (2 µg.) of plasmid pOW10 (prepared in Example 1D), in TE buffer, 2.5 µl. (100 mM) DTT, .5 µl. (1000 µg./ml.) BSA, 17 µl. water, 1 µl. (containing 4 New England Bio Lab Units) *Noc*I restriction enzyme and 2.5 µl. 10X reaction mix* were incubated at 37°C. for 1.5 hours. After the reaction was terminated by incubation at 65°C. for 10 minutes, the digested DNA was ethanol precipitated and then dissolved in 34.5 µl. of water.

B. *Bam*HI Digestion of Plasmid pMC1403

The desired digestion was carried out in substantial accordance with the teaching of Example 6A except that plasmid pMC1403 and *Bam*HI restriction enzyme and reaction mix, rather than plasmid pOW10 and *Nco*I restriction enzyme and reaction mix, were used. Plasmid pMC1403 was isolated from *E. coli* K12 BE904/pMC1403 (NRRL B—15213) in substantial accordance with the teaching of Example 1A—1. The digested DNA was ethanol precipitated and then dissolved in 34.5 µl. of water.

C. Klenow Fill-in of *Nco*I-Digested Plasmid pOW10 and *Bam*HI-Digested Plasmid pMC1403

The *Nco*I and *Bam*HI digests of Examples 6A and 6B were combined (69 µl.) and then incubated with 4 µl. each of dATP, dGTP, dCTP and TTP, 5 µl. (containing 10 units of DNA polymerase I large (Klenow) fragment† and 10 µl. of 10X Klenow buffer (.5M Tris-HCl, pH 7.2, .1M MgSO₄, 1 mM DTT) at 16°C. for 30 minutes. After the reaction was terminated by incubation at 65°C. for 10 minutes, the DNA was ethanol precipitated, extracted once with phenol and twice with chloroform:isoamyl alcohol (24:1) and then ethanol precipitated again. The Klenow filled-in DNA was dissolved in 20 µl. of water for subsequent *Eco*RI digestion.

D. *Eco*RI Digestion of the Klenow Filled-In *Nco*I and *Bam*HI Fragments

About 20 µl. (4 µg.) of the Klenow filled-in DNA, 5 µl. (100 mM)DTT, 5 µl. (1000 mg./ml.) BSA, 2 µl. (containing 10 New England Bio Lab Units) *Eco*RI restriction enzyme and 5 µl. (10X) reaction mix were incubated at 37°C. for 1 hour and then at 65°C. for 10 minutes. The resultant *Eco*RI-digested DNA was ethanol precipitated and then dissolved in 30 µl. of water.

---

* Reaction mix for *Nco*HI restriction enzyme was prepared with the following composition.
1500 mM NaCl
   60 mM Tris-HCl, pH 7.
   60 mM MgCl₂

† The Klenow fragment of DNA polymerase I can be obtained from the following source.
Boehringer-Mannheim Biochemicals
7941 Castleway Drive
Indianapolis, Indiana 46250

E.  Ligation of the *Eco*RI-Digested Klenow Filled-In *Nco*I and *Bam*HI Fragments and Construction of *E. coli* K12 BE904/pOW303

About 30 μl. of the *Eco*RI-digested Klenow filled-in fragments, 4 μl. (100 mM) DTT, 4 μl. (100 mM) ATP, 4 μl. 10X ligase buffer and 1 μl. (containing 10 New England Bio Lab Units) T4 DNA ligase were incubated at 16°C. for about 16 hours and then at 65°C. for 10 minutes. After cooling on ice about 15 μl. of the resultant ligated DNA, designated herein as plasmid pOW303, was used to transform *E. coli* K12 BE904 (NRRL B—15212). This was done in substantial accordance with the procedure of Example 1D except that plasmid pOW303, rather than plasmid pOW10, was used and 80 μg./ml. of ampicillin was used instead of tetracycline. In addition, the TY plates contained 40 μg./ml. of 5-bromo-4-chloro-3-indolyl-β-D-galactoside (x-gal) so that color could be used as a convenient assay of β-galactosidase activity. The resultant blue and ampicillin-resistant transformants constituted the desired *E. coli* K12 BE904/pOW303. The transformants were cultured, using conventional microbiological techniques, and were used for subsequent production and isolation of plasmid pOW303 in substantial accordance with the procedure of Example 1A—1. A restriction site map of plasmid pOW303 is presented in Figure 2 of the accompanying drawings.

Example 7
Construction of Plasmids pOW523 and pOW524 and *E. coli* K12 BE904/pOW523 and
*E. coli* K12 BE904/pOW524

The desired constructions were made in substantial accordance with the teaching of Examples 2D and 2E except that plasmid pOW303, rather than plasmid pOW10, and 80 μg./ml. of ampicillin was used instead of tetracycline. In addition, the TY plates contained 40 μg./ml. of x-gal so that color could be used as a convenient assay of β-galactosidase activity. Recombinant plasmids of two orientations result because the *Eco*RI-restricted DNA can be ligated in either direction. A restriction site map of each of plasmids pOW523 and pOW524 is presented in Figure 2 of the accompanying drawings.

Example 8
Construction of *Bacillus subtilis* MI112/pOW523 and B. subtilis MI112/pOW524

The desired constructions were made in substantial accordance with the teaching of Example 2C except that plasmids pOW523 and pOW524, rather than plasmid pHI—18, were used. In addition, the regeneration medium was modified so as to contain 40 μg./ml. of x-gal so that color would be used as a convenience assay of β-galactosidase activity. The resultant *Bacillus subtilis* MI112/pOW523 and *B. subtilis* MI112/pOW524 chloramphenicol-resistant and kanomycin-sensitive transformant colonies were isolated according to known procedures. The transformants produced dark blue colonies on x-gal-containing plates indicating that the gene that encoded the β-galactosidase activity was in the correct translational reading phase for direct expression. This was further confirmed by *in vitro* assay, which showed the presence of β-galactosidase activity in the transformants, and also by structural analysis of the plasmids. The desired *B. subtilis* MI112/pOW523 and *B. subtilis* MI112/pOW524 transformants were cultured and then conventionally identified by restriction enzyme and agarose gel electrophoretic analysis (Maniatis *et al.*, 1982), of the constitutive plasmids.

Example 9
Construction of Plasmids pOW529 and pOW530 and *E. coli* K12 BE904/pOW529 and
*E. coli* K12 BE904/pOW530

The desired constructions are made in substantial accordance with the teaching of Examples 2D and 2E except that plasmids pOW303 and pBS1, rather than plasmid pOW10 and pHI—18, are used and 80 μg./ml. of ampicillin is used instead of tetracycline. In addition, the TY plates contain 40 μg./ml. of x-gal so that color can be used as a convenient assay of β-galactosidase activity. Recombinant plasmids of two orientations result because the *Eco*RI-restricted DNA can be ligated in either direction. A restriction site map of each of plasmids pOW529 and pOW530 is presented in Figure 2 of the accompanying drawings.

Example 10
Construction of Bacillus subtilis MI112/pOW529 and *B. subtilis* MI112/pOW530

The desired constructions are made in substantial accordance with the teaching of Example 8 except that plasmids pOW529 and pOW530, rather than plasmids pOW523 and pOW524, are used. The transformants produce dark blue colonies on x-gal containing plates indicating that the gene that encodes the β-galactosidase activity is in the correct translational reading phase for direct expression. This is further confirmed by *in vitro* assay, which shows the presence of β-galactosidase activity in the transformants, and also by structural analysis of the plasmids. The desired *B. subtilis* MI112/pOW529 and *B. subtilis* MI112/pOW530 transformants are cultured and then conventionally identified by restriction enzyme and agarose gel electrophoretic analysis (Maniatis *et al.*, 1982), of the constitutive plasmids.

## Example 11
### Construction of the DNA Fragment

```
5' A G T G A G G T G G A T R C                3'
    I I I I I I I I I  I,I
3'           T C C A C C T A R¹G G T A C   5'
```

wherein R is C and R¹ is G

The desired construction involves the synthesis and 5' phosphorylation of oligonucleotides $T_3$ and $T_4$ shown below.

$T_3$  5'  AGTGAGGTGGATCC  3'
$T_4$  5'  CATGGGATCCACCT  3'

Oligonucleotides $T_3$ and $T_4$ were used to construct the desired DNA fragment having an *Nco*I sticky terminus adjacent to a *Bam*HI restriction site. The oligonucleotide synthesis was conventionally done by the modified phosphotriester method using fully protected deoxyribonucleotide building blocks. The desired construction was made in substantial accordance with the teaching of Example 1C.

## Example 12
### Construction of Plasmid pOW11 and *E. coli* K12 JA221/pOW11

The desired constructions are made in substantial accordance with the teaching of Example 1 except that the DNA fragment of Example 11, rather than the DNA fragment of Example 1C, is used. The restriction site map of plasmid pOW11 is the same, except for the aforementioned *Bam*HI site, as that shown for plasmid pOW10 in Figure 1 of the accompanying drawings.

## Example 13
### Construction of Plasmids pOW531 and pOW532 and *E. coli* K12 JA221/pOW531 and *E. coli* K12 JA221/pOW532

The desired constructions are made in substantial accordance with the teaching of Example 2 except that plasmid pOW11, rather than plasmid pOW10, is used. The respective restriction site map of each of plasmids pOW531 and pOW532 is the same, except for the aforementioned additional *Bam*HI site from the pOW11 fragment, as that shown for plasmids pOW525 and pOW526 in Figure 1 of the accompanying drawings.

## Example 14
### Construction of *Bacillus subtilis* MI112/pOW531 and *Bacillus subtilis* MI112/pOW532

The desired constructions are made in substantial accordance with the teaching of Example 3 except that plasmids pOW531 and pOW532, rather than plasmids pOW525 and pOW526, are used.

## Example 15
### Construction of Plasmids pOW533 and pOW534 and *E. coli* JA221/pOW533 and *E. coli* K12 JA221/pOW534

The desired constructions are made in substantial accordance with the teaching of Example 4 except that plasmid pOW11, rather than plasmid pOW10, is used. The respective restriction site map of each of plasmids pOW533 and pOW534 is the same, except for the aforementioned additional *Bam*HI site from the pOW11 fragment, as that shown for plasmids pOW527 and pOW528 in Figure 1 of the accompanying drawings.

## Example 16
### Construction of *Bacillus subtilis* MI112/pOW533 and *Bacillus subtilis* MI112/pOW534

The desired constructions are made in substantial accordance with the teaching of Example 5 except that plasmid pOW533 and pOW534, rather than plasmids pOW527 and pOW528, are used.

## Example 17
### Construction of Plasmid pOW310 and *E. coli* K12 BE904/pOW310

The desired constructions are made in substantial accordance with the teaching of Example 6 except that plasmid pOW11, rather than plasmid pOW10, is used. The restriction site map of plasmid pOW310 is the same, except for the aforementioned additional *Bam*HI site from the pOW11 fragment, as that shown for plasmid pOW303 in Figure 2 of the accompanying drawings.

## Example 18
### Construction of Plasmids pOW535 and pOW536 and *E. coli* K12 BE904/pOW535 and *E. coli* K12 BE904/pOW536

The desired constructions are made in substantial accordance with the teaching of Example 7 except that plasmid pOW310, rather than plasmid pOW303, is used. The restriction site map of each of plasmids pOW535 and pOW536 is the same, except for the aforementioned additional *Bam*HI site from the pOW310 fragment, as that presented for plasmids pOW523 and pOW524 in Figure 2 of the accompanying drawings.

Example 19
Construction of *Bacillus subtilis* MI112/pOW535 and *Bacillus subtilis* MI112/pOW536
The desired constructions are made in substantial accordance with the teaching of Example 8 except that plasmids pOW535 and pOW536, rather than plasmids pOW523 and pOW524, are used.

Example 20
Construction of Plasmids pOW537 and pOW538 and *E. coli* K12 BE904/pOW537 and *E. coli* K12 BE904/pOW538
The desired constructions are made in substantial accordance with the teaching of Example 9 except that plasmid pOW310, rather than plasmid pOW303, is used. The restriction site map of each of plasmids pOW537 and pOW538 is the same, except for the aforementioned additional *Bam*HI site from the pOW310 fragment, as that presented for plasmids pOW529 and pOW530 in Figure 2 of the accompanying drawings.

Example 21
Construction of *Bacillus subtilis* MI112/pOW537 and *Bacillus subtilis* MI112/pOW538
The desired constructions are made in substantial accordance with the teaching of Example 10 except that plasmid pOW537 and pOW538, rather than plasmids pOW529 and pOW530, are used.

**Claims**

1. A ribosome binding site-containing DNA sequence which is

$$
\begin{array}{l}
5'\ \ A\ G\ T\ G\ A\ G\ G\ T\ G\ G\ A\ T\ R\ C \qquad\qquad 3' \\
\qquad\quad |\ \ |\ \ |\ \ |\ \ |\ \ |\ \ |\ \ |\ \ |_1| \\
3' \qquad\qquad T\ C\ C\ A\ C\ C\ T\ A\ R^1 G\ G\ T\ A\ C\ \ 5'
\end{array}
$$

wherein
A is deoxyadenyl,
G is deoxyguanyl,
C is deoxycytosyl,
T is thymidyl,
R is G or C, and
$R^1$ is G or C,
subject to the limitation that R and $R^1$ are not simultaneously the same deoxyribonucleotide;
2. A recombinant DNA expression vector which comprises
(a) the ribosome binding site-containing DNA sequence of Claim 1,
(b) the *veg* promoter of *Bacillus subtilis*;
(c) a gene that encodes a functional polypeptide,
(d) an *E. coli* replicon, and optionally
(e) a *Bacillus* replicon or
(f) a *Bacillus* replicon and a *Streptomyces* replicon;
subject to the limitation that the vector is selectable and that the *veg* promoter and the DNA sequence of Claim 1 direct transcription and expression of the gene in a host cell transformed by the vector.
3. The vector of Claim 2 which is a plasmid.
4. The vector of Claim 2 or 3 wherein the functional polypeptide encoded by the gene is human pre-proinsulin, human proinsulin, human insulin A-chain, human insulin B-chain, non-human insulin, human growth hormone, non-human growth hormone, bovine growth hormone, porcine growth hormone, human interferon, non-human interferon, viral antigen, urokinase, any peptide hormone or enzyme.
5. The vector of Claim 2, 3 or 4 in which R is G and $R^1$ is C in the ribosome binding site-containing DNA sequence.
6. The vector of Claim 5 which comprises the human pre-proinsulin gene contained in the ~4kb *Eco*RI-*Nco*I fragment of plasmid pOW601 (NRRL B—15259), a modified *veg* promoter which lies on the *Eco*RI-*Sfa*NI restriction fragment of pMS480 (NRRL B—15258) and is designated plasmid pOW10.
7. The vector of Claim 5 which comprises the human pre-proinsulin gene contained in the ~4kb *Eco*RI-*Nco*I restriction fragment of plasmid pOW601 (NRRL B—15259) and a *Bacillus* replicon.
8. The vector of Claim 7 which is plasmid pOW525 as shown in Figure 1(1).
9. The vector of Claim 7 which is plasmid pOW526 as shown in Figure 1(2).
10. The vector of Claim 7 which also comprises a *Streptomyces* replicon.
11. The vector of Claim 10 which is plasmid pOW527 as shown in Figure 1(3).
12. The vector of Claim 10 which is plasmid pOW528 as shown in Figure 1(4).

EP 0 116 411 B1

13. The vector of Claim 5 which comprises the portion of the *lacZ* gene contained in the *Bam*HI digest of plasmid pMC1403 (NRRL B—15213), a modified *veg* promoter which lies on the *Eco*RI-*Sfa*NI restriction fragment of pMS480 (NRRL B—15258) and is designated plasmid pOW303.

14. The vector of Claim 5 which comprises the portion of the *lacZ* gene contained in the *Bam*HI digest of plasmid pMC1403 (NRRL B—15213) and a *Bacillus* replicon.

15. The vector of Claim 14 which is plasmid pOW523 as shown in Figure 2(1).

16. The vector of Claim 14 which is plasmid pOW524 as shown in Figure 2(2).

17. The vector of Claim 14 which also comprises a *Streptomyces* replicon.

18. The vector of Claim 17 which is plasmid pOW529 as shown in Figure 2(3).

19. The vector of Claim 17 which is plasmid pOW530 as shown in Figure 2(4).

20. The vector of Claim 2, 3 or 4 in which R is C and $R^1$ is G in the ribosome binding site-containing DNA sequence.

21. The vector of Claim 20 which comprises the human pre-proinsulin gene contained in the ~4kb *Eco*RI-*Nco*I fragment of plasmid pOW601 (NRRL B—15259), a modified *veg* promoter which lies on the *Eco*RI-*Sfa*NI restriction fragment of pMS480 (NRRL B—15258) and is designated pOW11.

22. The vector of Claim 20 which comprises the human pre-proinsulin gene contained in the ~4kb *Eco*RI-*Nco*I restriction fragment of plasmid pOW601 (NRRL B—15259) and a *Bacillus* replicon.

23. The vector of Claim 22 which is plasmid pOW531 as shown in Figure 1(1) with the modification that the *Eco*RI-*Eco*RI insert contains an additional *Bam*HI restriction site.

24. The vector of Claim 22 which is plasmid pOW532 as shown in Figure 1(2) with the modification that the *Eco*RI-*Eco*RI insert contains an additional *Bam*HI restriction site.

25. The vector of Claim 22 which also comprises a *Streptomyces* replicon.

26. The vector of Claim 25 which is plasmid pOW533 as shown in Figure 1(3) with the modification that the *Eco*RI-*Eco*RI insert contains an additional *Bam*HI restriction site.

27. The vector of Claim 25 which is plasmid pOW534 as shown in Figure 1(4) with the modification that the *Eco*RI-*Eco*RI insert contains an additional *Bam*HI restriction site.

28. The vector of Claim 20 which comprises the portion of the *lacZ* gene contained in the *Bam*HI digest of plasmid pMC1403 (NRRL B—15213), a modified *veg* promoter which lies on the *Eco*RI-*Sfa*NI restriction fragment of pMS480 (NRRL B—15258) and is designated plasmid pOW310.

29. The vector of Claim 20 which comprises the portion of the *lacZ* gene contained in the *Bam*HI digest of plasmid pMC1403 (NRRL B—15213) and a *Bacillus* replicon.

30. The vector of Claim 29 which is plasmid pOW535 as shown in Figure 2(1) with the modification that the *Eco*RI-*Eco*RI insert contains an additional *Bam*HI restriction site.

31. The vector of Claim 29 which is plasmid pOW536 as shown in Figure 2(2) with the modification that the *Eco*RI-*Eco*RI insert contains an additional *Bam*HI restriction site.

32. The vector of Claim 29 which also comprises a *Streptomyces* replicon.

33. The vector of Claim 32 which is plasmid pOW537 as shown in Figure 2(3) with the modification that the *Eco*RI-*Eco*RI insert contains an additional *Bam*HI restriction site.

34. The vector of Claim 32 which is plasmid pOW538 as shown in Figure 2(4) with the modification that the *Eco*RI-*Eco*RI insert contains an additional *Bam*HI restriction site.

35. A transformed host cell which comprises the vector of any of Claims 2 to 34.

36. The host cell of Claim 35 which is a *Bacillus* preferably of species *subtilis,* comprising the vector of any of Claims 7 to 12, 14 to 19, 22 to 27 or 29 to 34.

37. The host cell of Claim 36 which is *Bacillus subtilis* MI112.

38. The host cell of Claim 34 which is *E. Coli.*

39. The host cell of Claim 38 which is *E. Coli* K12 JA221 comprising the vector of any of Claims 6 to 19.

40. The host cell of Claim 38 which is *E. Coli* K12 BE904 comprising the vector of any of Claims 21 to 34.

41. A process for preparing a recombinant DNA expression vector which comprises ligating

1) the ribosome binding site-containing DNA sequence of Claim 1

2) the *veg* promoter of *Bacillus subtilis,* and

3) a gene that encodes a functional polypeptide, subject to the limitation that the vector is selectable and that the *veg* promoter and the DNA sequence of Claim 1 direct transcription and expression of the gene in a host cell transformed by the vector.

42. A method for producing a functional polypeptide in *Bacillus* wherein the polypeptide is secreted into the culture medium, which comprises transforming a *Bacillus* host cell with the recombinant expression vector of any of Claims 7 to 12, 14 to 19, 22 to 27 or 29 to 34 and culturing said transformed *Bacillus* host cell under growth conditions.

19

**Patentansprüche**

1. Eine eine Ribosomenbindungsstelle enthaltende DNA-Sequenz, nämlich

$$5' \quad \text{A G T G A G G T G G A T R C} \qquad \qquad 3'$$
$$\qquad \quad | \; | \; | \; | \; | \; | \; | \; | \; | \; |$$
$$3' \qquad \qquad \quad \text{T C C A C C T A R}^1 \text{G G T A C} \quad 5'$$

worin

A Desoxyadenyl bedeutet,
G Desoxyguanyl bedeutet,
C Desoxycytosyl bedeutet,
T Thymidyl bedeutet,
R die Bedeutung G oder C hat und
$R^1$ die Bedeutung G oder C hat,
mit der Einschränkung, dass R und $R^1$ nicht gleichzeitig das gleiche Desoxyribonucleotid bedeuten.

2. Rekombinanter DNA-Expressionsvektor, enthaltend
(a) die eine Ribosomenbindungsstelle enthaltende DNA-Sequenz nach Anspruch 1,
(b) den veg-Promotor von Bacillus subtilis,
(c) ein Gen, das für ein funktionelles Polypeptid kodiert,
(d) ein E. coli-Replikon und gegebenefalls
(e) ein Bacillus-Replikon oder
(f) ein Bacillus-Replikon und ein Streptomyces-Replikon,
mit der Einshräkung, dass der Vektor selektierbar ist und dass der veg-Promotor und die DNA-Sequenz nach Anspruch 1 die Transkription und Expression des Gens in eine mit dem Vektor transformierte Wirtszelle dirigieren.

3. Vektor nach Anspruch 2, bei dem es sich um ein Plasmid handelt.

4. Vektor nach Anspruch 2 oder 3, wobei es sich bei dem funktionellen Polypeptid, für das das Gen kodiert, um humanes Präproinsulin, humanes Proinsulin, humane Insulin A-Kette, humane Insulin B-Kette, nicht-humanes Insulin, humanes Wachstumshormon, nicht-humanes Wachstomshormon, Rinderwachstumshormon, Schweinewachstumshormon, humanes Interferon, nicht-humanes Interferon, virales Antigen, Urokinase, beliebige Peptidhormone oder Enzyme handelt.

5. Vektor nach Anspruch 2, 3 oder 4, bei dem in der die Ribosomenbindungsstelle enthaltenden DNA-Sequenz R die Bedeutung G und $R^1$ die Bedeutung C hat.

6. Vektor nach Anspruch 5, der das im -4 kb-EcoRI-NcoI-Fragment des Plasmids pOW601 (NRRL B—15259) enthaltene humane Präproinsulin-Gen und einen modifizierten veg-Promotor, der am EcoRI-SfaNI-Restriktionsfragment von pMS480 (NRRL B—15258) liegt, enthält und als Plasmid pOW10 bezeichnet wird.

7. Vektor nach Anspruch 5, der das im -4 kb-EcoRI-NcoI-Restriktionsfragment von Plasmid pOW601 (NRRL B—15259) enthaltene humane Präproinsulin-Gen und ein Bacillus-Replikon enthält.

8. Vektor nach Anspruch 7, bei dem es sich um das in Fig. 1(1) gezeigte Plasmid pOW525 handelt.

9. Vektor nach Anspruch 7, bei dem es sich um das in Fig. 1(2) gezeigte Plasmid pOW526 handelt.

10. Vektor nach Anspruch 7, der zusätzlich ein Streptomyces-Replikon enthält.

11. Vektor nach Anspruch 10, bei dem es sich um das in Fig. 1(3) gezeigte Plasmid pOW527 handelt.

12. Vektor nach Anspruch 10, bei dem es sich um das in Fig. 1(4) gezeigte Plasmid pOW528 handelt.

13. Vektor nach Anspruch 5, der den im BamHI-Verdauungsprodukt des Plasmids pMC1403 (NRRL B—15213) enthaltenen Bereich des lac Z-Gens und einen modifizierten veg-Promotor, der am EcoRI-SfaNI-Restriktionsfragment von pMS480 (NRRL B—15258) liegt, enthält und als Plasmid pOW303 bezeichnet wird.

14. Vektor nach Anspruch 5, der den im *Bam*HI-Verdauungsprodukt des Plasmids pMC1403 (NRRL B—15213) enthaltenen Bereich des lac Z-Gens und ein Bacillus-Replikon enthält.

15. Vektor nach Anspruch 14, bei dem es sich um das in Fig. 2(1) gezeigte Plasmid pOW523 handelt.

16. Vektor nach Anspruch 14, bei dem es sich um das in Fig. 2(2) gezeigte Plasmid pOW524 handelt.

17. Vektor nach Anspruch 14, der zusätzlich ein Streptomyces-Replikon enhält.

18. Vektor nach Anspruch 17, bei dem es sich um das in Fig. 2(3) gezeigte Plasmid pOW529 handelt.

19. Vektor nach Anspruch 17, bei dem es sich um das in Fig. 2(4) gezeigte Plasmid pOW530 handelt.

20. Vektor nach Anspruch 2, 3 oder 4, bei dem in der die Ribosomenbindungsstelle enthaltenden DNA-Sequenz R die Bedeutung C und $R^1$ die Bedeutung G hat.

21. Vektor nach Anspruch 20, der das im -4 kb-EcoRI-NcoI-fragment des Plasmids pOW601 (NRRL B—15259) enthaltene humane Präproinsulin-Gen und einen modifizierten veg-Promotor, der am EcoRI-SfaNI-Restriktionsfragment von pMS480 (NRRL B—15258) liegt, enthält und als Plasmid pOW11 bezeichnet wird.

22. Vektor nach Anspruch 20, der das im -4 kb-EcoRI-NcoI-Restriktionsfragment des Plasmids pOW601 (NRRL B—15259) enthaltene humane Präproinsulin-Gen und ein Bacillus-Replikon enthält.

23. Vektor nach Anspruch 22, bei dem es sich um das in Fig. 1(1) gezeigte Plasmid pOW531 handelt, mit der Abänderung, dass das EcoRI-EcoRI-Insertionsprodukt eine zusätzliche BamHI-Restriktionsstelle enthält.

24. Vektor nach Anspruch 22, bei dem es sich um das in Fig. 1(2) gezeigte Plasmid pOW532 handelt, mit der Abänderung, dass das EcoRI-EcoRI-Insertionsprodukt eine zusätzliche BamHI-Restriktionsstelle enthält.

25. Vektor nach Anspruch 22, der zusätzlich ein Streptomyces-Replikon enthält.

26. Vektor nach Anspruch 25, bei dem es sich um das in Fig. 1(3) gezeigte Plasmid pOW533 handelt, mit der Abänderung, dass das EcoRI-EcoRI-Insertionsprodukt eine zusätzliche BamHI-Restriktionsstelle enthält.

27. Vektor nach Anspruch 25, bei dem es sich um das in Fig. 1(4) gezeigte Plasmid pOW534 handelt, mit der Abänderung, dass das EcoRI-EcoRI-Insertionsprodukt eine zusätzliche BamHI-Restriktionsstelle enthält.

28. Vektor nach Anspruch 20, der den im BamHI-Verdauungsprodukt des Plasmids pMC1403 (NRRL B—15213) enthaltenen Bereich des lac Z-Gens und einen mofizierten veg-promotor, der am EcoRI-SfaNI-Restriktionsfragment von pMS480 (NRRL—B—15258) liegt, enthält und als Plasmid pOW310 bezeichnet wird.

29. Vektor nach Anspruch 20, der den im BanHI-Verdauungsprodukt des Plasmids pMC1403 (NRRL B—15213) enthaltenen Bereich des lac Z-Gens und ein Bacillus-Replikon enthält.

30. Vektor nach Anspruch 29, bei dem es sich um das in Fig. 2(1) gezeigte Plasmid pOW535 handelt, mit der Abänderung, dass das EcoRi-EcoRI-Insertionsprodukt eine zusätzliche BamHI-Restriktionsstelle enthält.

31. Vektor nach Anspruch 29, bei dem es sich um das in Fig. 2(2) gezeigte Plasmid pOW536 handelt, mit der Abänderung, dass das EcoRI-EcoRI-Insertionsprodukt eine zusätzliche BamHI-Restriktionsstelle enthält.

32. Vektor nach Anspruch 29, der zusätzlich ein Streptomyces-Replikon enthält.

33. Vektor nach Anspruch 32, bei dem es sich um das in Fig. 2(3) gezeigte Plasmid pOW537 handelt, mit der Abänderung, dass das EcoRI-EcoRI-Insertionsprodukt eine zusätzliche BamHI-Restriktionsstelle enthält.

34. Vektor nach Anspruch 32, bei dem es sich um das in Fig. 2(4) gezeigte Plasmid pOW538 handelt, mit der Abänderung, dass das EcoRI-EcoRI-Insertionsprodukt eine zusätzliche BamHI-Restriktionsstelle enthält.

35. Transformierte Wirtszelle, die den Vektor nach einem der Ansprüche 2 bis 34 enthält.

36. Wirtszelle nach Anspruch 35, bei der es sich um Bacillus, vorzugsweise der Spezies subtilis, handelt, enthaltend den Vektor nach einem der Ansprüche 7 bis 12, 14 bis 19, 22 bis 27 oder 29 bis 34.

37. Virtszelle nach Anspruch 36, bei der es sich um Bacillus subtilis MI112 handelt.

38. Wirtszelle nach Anspruch 34, bei der es sich um E. coli handelt.

39. Wirtszelle nach Anspruch 38, bei der es sich um E. coli K12 JA221 mit einem Gehalt an dem Vektor nach einem der Ansprüche 6 bis 19 handelt.

40. Wirtszelle nach Anspruch 38, bei der es sich um E. coli K12 BE904 mit einem Gehalt an dem Vektor nach einem der Ansprüche 21 bis 34 handelt.

41. Verfahren zur Herstellung eines rekombinanten DNA-Expressionsvektors, umfassend die Verknüpfung

1) der die Ribosomenbindungsstelle enthaltenden DNA-Sequenz von Anspruch 1,

2) des veg-Promotors von Bacillus subtilis und

3) eines Gens, das für ein funktionelles Polypeptid kodiert,

mit der Einschränkung, dass der Vektor selektierbar ist und dass der veg-Promotor und die DNA-Sequenz von Anspruch 1 die Transkription und die Expression des Gens in einer mit dem Vektor transformierten Wirtszelle dirigieren.

42. Verfahren zur Herstellung eines funktionellen Polypeptids in Bacillus, wobei das Polypeptid in das Kulturmedium sekretiert wird, umfassend die Transformation einer Bacillus-Wirtszelle mit dem rekombinanten Expressionsvektor nach einem der Ansprüche 7 bis 12, 14 bis 19, 22 bis 27 oder 29 bis 34 und die Züchtung der transformierten Bacillus-Wirtszelle unter Wachstumsbedingungen.

## Revendications

1. Séquence d'ADN contenant un site de liaison ribosomique, a savoir

$$5' \ A \ G \ T \ G \ A \ G \ G \ T \ G \ G \ A \ T \ R \ C \qquad 3'$$
$$\qquad\qquad | \ \ | \ \ | \ \ | \ \ | \ \ | \ \ | \ \ | \ \ | \ |$$
$$3' \qquad\qquad T \ C \ C \ A \ C \ C \ T \ A \ R^1 G \ G \ T \ A \ C \qquad 5'$$

A représente un groupe désoxyadényle,

G représente un groupe désoxyguanyle,

C représente un groupe désoxycytosyle,

T représente un groupe thymidyle,

R représente G ou C, et

$R^1$ représente G ou C,

avec cette réserve que R et $R^1$ ne représentent pas simultanément le même désoxyribonucléotide.

2. Vecteur d'expression d'ADN recombinant comprenant:

(a) la séquence d'ADN contenant le site de liaison ribosomique selon la revendication 1,

(b) le promoteur *veg* de *Bacillus subtilis;*

(c) un gène qui encode un polypeptide fonctionnel;

(d) un réplicon *E. coli,* et éventuellement

(e) un réplicon *Bacillus* ou

(f) un réplicon *Bacillus* et un réplicon *Streptomyces;*

avec cette réserve que le vecteur soit sélectionnable et que le promoteur *veg* et la séquence d'ADN selon la revendication 1 régissent la transcription et l'expression du gène dans une cellule hôte transformée par le vecteur.

3. Vecteur selon la revendication 2, ce vecteur étant un plasmide.

4. Vecteur selon la revendication 2 ou 3 dans lequel le polypeptide fonctionnel encode par le gène représente la pré-proinsuline humaine, la proinsuline humaine, la chaîne A de l'insuline humaine, la chaîne B de l'insuline humaine, l'insuline non humaine, l'hormone humaine de croissance, l'hormone non humaine de croissance, l'hormone bovine de croissance, l'hormone porcine de croissance, l'interféron humain, l'interféron non humain, les antigènes viraux, l'urokinase, n'importe quelle hormone peptidique ou n'importe quelle enzyme.

5. Vecteur selon la revendication 2, 3 ou 4, dans lequel R représenté G et $R^1$ représente C dans la séquence d'ADN contenant un site de liaison ribosomique.

6. Vecteur selon la revendication 5, celui-ci comprenant le gène de la pré-proinsuline humaine contenu dans le fragment *Eco*RI-*Nco*I d'environ 4 kb du plasmide pOW601 (NRRL B—15259), un promoteur *veg* modifié qui se trouve sur le fragment de restriction *Eco*RI-*Sfa*NI de pMS480 (NRRL B—15258) et qui est désigné par l'expression "plasmide pOW10".

7. Vecteur selon la revendication 5, ce vecteur comprenant le gène de la pré-proinsuline humaine, contenu dans le fragment de restriction *Eco*RI-*Nco*I d'environ 4 kb du plasmide pOW601 (NRRL B—15259), et un réplicon *Bacillus.*

8. Vecteur selon la revendication 7, celui-ci étant le plasmide pOW525 illustré en figure 1(1).

9. Vecteur selon la revendication 7, celui-ci étant le plasmide pOW526 illustré en figure 1(2).

10. Vecteur selon la revendication 7, celui-ci comprenant également un réplicon *Streptomyces.*

11. Vecteur selon la revendication 10, celui-ci étant le plasmide pOW527 illustre en figure 1(3).

12. Vecteur selon la revendication 10, celui-ci étant le plasmide pOW528 illustré en figure 1(4).

13. Vecteur selon la revendication 5, celui-ci comprenant la portion du gène *lacZ* contenue dans le produit de digestion par *Bam*HI du plasmide pMC1403 (NRRL B—15213), un promoteur *veg* modifié qui se trouve sur le fragment de restriction *Eco*RI-*Sfa*NI de pMS480 (NRRL B—15258) et qui est désigné par l'expression "plasmide pOW303".

14. Vecteur selon la revendication 5, celui-ci comprenant la portion du gène *lacZ* contenue dans le produit de digestion par *Bam*HI du plasmide pMC1403 (NRRL B—15213), et un réplicon *Bacillus.*

15. Vecteur selon la revendication 14, celui-ci étant le plasmide pOW523 illustré en figure 2(1).

16. Vecteur selon la revendication 14, celui-ci étant le plasmide pOW524 illustré en figure 2(2).

17. Vecteur selon la revendication 14, celui-ci comprenant également un réplicon *Streptomyces.*

18. Vecteur selon la revendication 17, celui-ci étant le plasmide pOW529 illustré en figure 2(3).

19. Vecteur selon la revendication 17, celui-ci étant le plasmide pOW530 illustré en figure 2(4).

20. Vecteur selon la revendication 2, 3 ou 4, dans lequel R représente C et $R^1$ représente G dans la sequence d'ADN contenant un site de liaison ribosomique.

21. Vecteur selon la revendication 20, ce vecteur comprenant le gène de la pré-proinsuline humaine contenu dans le fragment *Eco*RI-*Sfa*NI d'environ 4 kb du plasmide pOW601 (NRRL B—15259), un promoteur *veg* modifié qui se trouve sur le fragment de restriction *Eco*RI-*Nco*I de pMS480 (NRRL B—15258), le vecteur étant désigné par l'expression "pOW11".

22. Vecteur selon la revendication 20, ce vecteur comprenant le gène de la pré-proinsuline humaine contenu dans le fragment *Eco*RI-*Nco*I d'environ 4 kb du plasmide pOW601 (NRRL B—15259) et un réplicon *Bacillus.*

23. Vecteur selon la revendication 22, ce vecteur étant le plasmide pOW531 tel qu'illustré en figure 1(1), avec cette modification que le produit d'insertion *Eco*RI-*Eco*RI contient un site de restriction *Bam*HI supplémentaire.

24. Vecteur selon la revendication 22, ce vecteur étant le plasmide pOW532 tel qu'illustré en figure 1(2), avec cette modification que le produit d'insertion *Eco*RI-*Eco*RI contient un site de restriction *Bam*HI supplémentaire.

25. Vecteur selon la revendication 22, ce vecteur comprenant également un réplicon *Streptomyces.*

26. Vecteur selon la revendication 25, ce vecteur étant le plasmide pOW533 tel qu'illustré en figure 1(3), avec cette modification que le produit d'insertion *Eco*RI-*Eco*RI contient un site de restriction *Bam*HI supplémentaire.

27. Vecteur selon la revendication 25, ce vecteur étant le plasmide pOW534 tel qu'illustré en figure 1(4), avec cette modification que le produit d'insertion *Eco*RI-*Eco*RI contient un site de restriction *Bam*HI supplémentaire.

28. Vecteur selon la revendication 20, ce vecteur comprenant la portion du gène *lacZ* contenue dans le produit de digestion par *Bam*HI du plasmide pMC1403 (NRRL B—15213), un promoteur *veg* modifie qui se trouve sur le fragment de restriction *Eco*RI-*Sfa*NI de pMS480 (NRRL B—15258), le vecteur étant désigné par l'expression "pOW310".

## EP 0 116 411 B1

29. Plasmide selon la revendication 20, ce plasmide comprenant la portion du gène *lac*Z contenue dans le produit de digestion par *Bam*HI du plasmide pMC1403 (NRRL B—15213) et un réplicon *Bacillus.*

30. Vecteur selon la revendication 29, ce vecteur étant le plasmide pOW535 tel qu'illustré en figure 2(1), avec cette modification que le produit d'insertion *Eco*RI-*Eco*RI contient un site de restriction *Bam*HI supplémentaire.

31. Vecteur selon la revendication 29, ce vecteur étant le plasmide pOW536 tel qu'illustré en figure 2(2), avec cette modification que le produit d'insertion *Eco*RI-*Eco*RI contient un site de restriction *Bam*HI supplémentaire.

32. Vecteur selon la revendication 29, ce vecteur comprenant également un réplicon *Streptomyces.*

33. Vecteur selon la revendication 32, ce vecteur étant le plasmide pOW537 tel qu'illustré en figure 2(3), avec cette modification que le produit d'insertion *Eco*RI-*Eco*RI contient un site de restriction *Bam*HI supplémentaire.

34. Vecteur selon la revendication 32, ce vecteur étant le plasmide pOW538 tel qu'illustré en figure 2(4), avec cette modification que le produit d'insertion *Eco*RI-*Eco*RI contient un site de restriction *Bam*HI supplémentaire.

35. Cellule hôte transformée cette cellule comprenant le vecteur de l'une quelconque des revendications 2 à 34.

36. Cellule hôte selon la revendication 35, cette cellule étant une cellule *Bacillus,* de préférence, de l'espèce *subtilis,* et comprenant le vecteur selon l'une quelconque des revendications 7 à 12, 14 à 19, 22 à 27 ou 29 à 34.

37. Cellule hôte selon la revendication 36, cette cellule étant *Bacillus subtilis* MI112.

38. Cellule hôte selon la revendication 34, cette cellule étant *E. coli.*

39. Cellule hôte selon la revendication 38, cette cellule étant *E. coli* K12 JA221 comprenant le vecteur de l'un quelconque des vecteurs des revendications 6 à 19.

40. Cellule hôte selon la revendication 38, cette cellule étant *E. coli* K12 BE904 comprenant le vecteur de l'une quelconque des revendications 21 à 34.

41. Procédé pour la préparation d'un vecteur d'expression d'ADN recombinant, ce procédé consistant à ligaturer

1) la séquence d'ADN contenant un site de liaison ribosomique selon la revendication 1;

2) le promoteur *veg* de *Bacillus subtilis,* et

3) un gène qui encode un polypeptide fonctionel,

avec cette réserve que le vecteur soit sélectionnable et que le promoteur *veg* et la séquence d'ADN selon la revendication 1 régissent la transcription et l'expression du gène dans une cellule hôte transformée par le vecteur.

42. Procédé pour la production d'un polypeptide fonctionnel dans *Bacillus* procédé selon lequel le polypeptide est sécrété au sein du milieu de culture, ce procédé consistant à transformer une cellule hôte Bacillus avec le vecteur d'expression recombinant selon l'une quelconque des revendications 7 à 12, 14 à 19, 22 à 27 ou 29 à 34 et à cultiver cette cellule hôte *Bacillus* transformée, dans des conditions de croissance.

23

# FIG. I
## Restriction Site Map of Plasmids pOW 525, pOW 526, pOW 527 and pOW 528

1. pOW 525 (~8.4 kb)
2. pOW 526 (~8.4 kb)
3. pOW 527 (~12.8 kb)
4. pOW 528 (~12.8 kb)

# FIG. 2
# Restriction Site Map of Plasmids pOW 523, pOW 524, pOW 529 and pOW530

pOW 303

Bam HI
Nco I

Eco RI

Eco RI                                    Eco RI

1.    Eco RI                                              Eco RI

         Pvu II          Bst NI
2.    Eco RI                                              Eco RI
         Xba I      Bam HI

                        Pvu II
3.    Eco RI                                              Eco RI
         Bst NI      Bam HI    Xba I

                Xho I        Cla I
4.    Eco RI                                              Eco RI
         Bam HI    Bam HI    Bam HI

    Bam HI    Bam HI    Bam HI
         Cla I        Xho I

1. pOW 523 ～ 14.2 kb
2. pOW 524 ～ 14.2 kb
3. pOW 529 ～ 18.6 kb
4. pOW 530 ～ 18.6 kb

# FIG. 3
## Synthesis Procedure for Fragment T₁

EP 0 116 411 B1

# FIG. 4
## Restriction Site Map of Plasmids
## pEL 107 and pEL 105

pEL107

pEL105

4

# FIG. 5
## Restriction Site Map of Plasmids pBS 1 and pBS 3

pBS 1

pBS 3